# EUROPEAN PATENT SPECIFICATION

(11) **EP 3 295 340 B1**
(45) Date of publication and mention of the grant of the patent: **30.12.2020**
(21) Application number: 16722661.2
(22) Date of filing: 12.05.2016
(51) Int. Cl.: G16H 50/50, G09B 23/28, A61M 1/36, A61M 1/14, A61M 1/16, G09B 23/30

(54) **A CLINICAL PARAMETER CALCULATION-SIMULATION-MONITORING SYSTEM**
SYSTEM ZUR BERECHNUNG, SIMULATION UND ÜBERWACHUNG KLINISCHER PARAMETER
SYSTÈME DE CALCUL, DE SIMULATION ET DE SURVEILLANCE DE PARAMÈTRES CLINIQUES

(30) Priority: 13.05.2015 US 201562160689 P
(43) Date of publication of application: 21.03.2018
(73) Proprietor: Maquet Cardiopulmonary GmbH, 76437 Rastatt (DE)
(72) Inventor: JONES, Dwayne K., Ottawa, Ontario K1T 2P7 (CA); NIGRONI, Paul A., Wanaque, New Jersey 07465 (US); ASHTON, Wesley Scott, Bloomingdale, NJ 07403 (US)
(74) Representative: Zacco GmbH
(86) International application number: PCT/EP2016/060786
(87) International publication number: WO 2016/180953

(56) References cited:
- WO-A1-2015/047927
- US-A- 5 931 160
- US-A1- 2008 017 194
- US-A1- 2014 073 900
- US-A1- 2014 099 617
- US-A1- 2015 105 642
- REES S E ET AL: "Using physiological models and decision theory for selecting appropriate ventilator settings", JOURNAL OF CLINICAL MONITORING AND COMPUTING, KLUWER ACADEMIC PUBLISHERS, DO, vol. 20, no. 6, 15 September 2006 (2006-09-15), pages 421-429, XP019451338, ISSN: 1573-2614, DOI: 10.1007/S10877-006-9049-5

## Description

### FIELD OF THE DISCLOSURE

The present disclosure pertains broadly to the field of patient monitoring systems, such as may be used during cardiac bypass surgeries and the like. Broadly, the present disclosure pertains to a clinical parameter calculation-simulation-monitoring system, such as may be used to calculate, simulate and/or monitor complicated clinical parameter, such as those clinical parameters that are functions of multiple measured variables. For example, the present disclosure pertains to an oxygen delivery and consumption calculation-simulation-monitoring system that facilitates the calculation, simulation, and/or monitoring of oxygen delivery and oxygen consumption for a patient during cardiac bypass surgeries, and like surgical and/or medical situations in which the calculation, simulation, and/or monitoring of oxygen delivery and oxygen consumption is desired. The present disclosure also pertains to a perfusion calculation-simulation-monitoring system that facilitates the calculation, simulation and/or monitoring of a patient's hematocrit or hemoglobin values during cardiac bypass surgeries, and like surgical and/or medical situations in which the calculation, simulation and/or monitoring of a patient's perfusion status is required.

### BACKGROUND OF THE DISCLOSURE

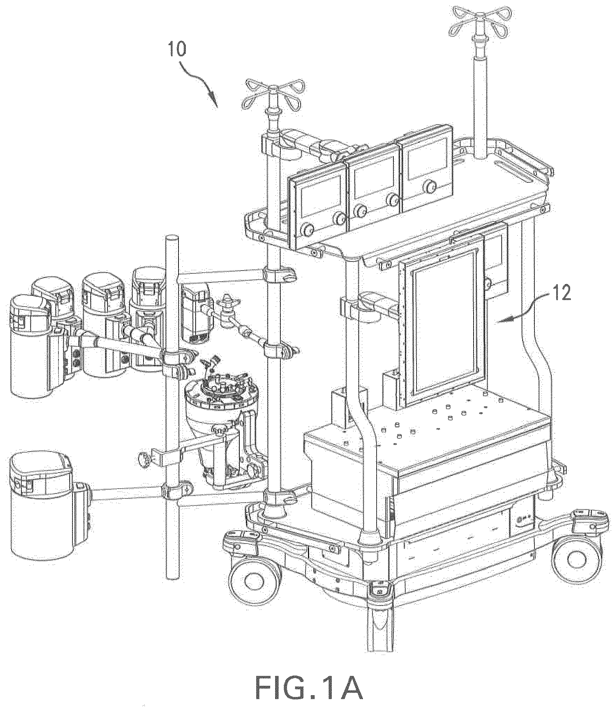

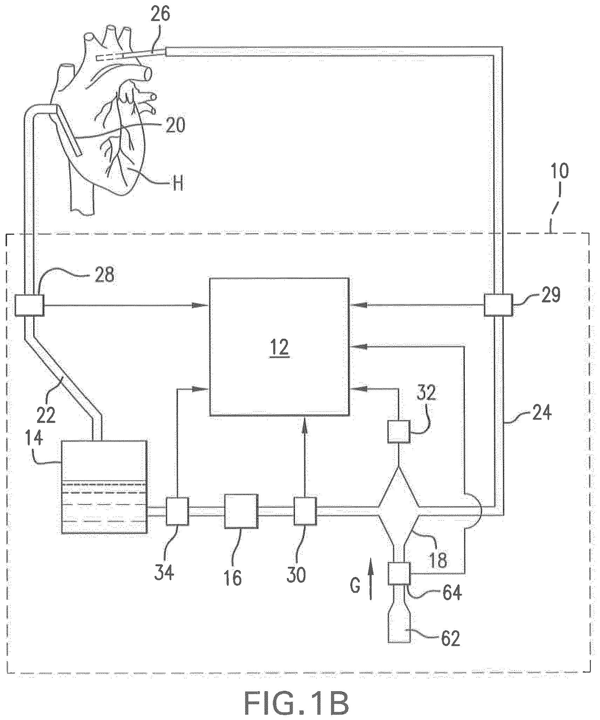

Open heart surgery may be regarded as one of the most important medical advances in the 20th century, and cardiopulmonary bypass has been key to the development of open heart surgery. The term cardiopulmonary bypass describes technology in which the circumvention of native heart and lungs is achieved with the use of extracorporeal devices. Examples of extracorporeal devices that may be used to circumvent a patient's native heart and/or lungs to provide mechanical means for pumping and oxygenating blood include cardiopulmonary bypass machines and extracorporeal membrane oxygenation (ECMO) machines. For an example of a conventional cardiopulmonary bypass circuit using a membrane oxygenator with integral hard-shell reservoir, one may consult FIG. 1 of Chapter 5 of Cardiopulmonary Bypass: Principles and Practices, Third Edition, Glenn P. Gravlee et al. (Eds.)(2008).

Employing a cardiopulmonary bypass machine to replace the function of a patient's heart and lungs requires constant monitoring of the patient's perfusion, oxygen delivery, and physiological parameters to ensure that the patient's oxygen consumption needs are met by the oxygen delivery provided by the cardiopulmonary bypass machine. If the cardiopulmonary bypass machine is not operated optimally to provide the patient with adequate mechanical perfusion, and/or to provide an oxygen delivery that surpasses the patient's oxygen consumption needs, then increased morbidity and mortality may result from tissue hypoxia due to inadequate oxygen delivery to the patient's tissues and anaerobic metabolism.

Scientific studies have established that insufficient oxygen delivery during cardiopulmonary bypass, due either to excessive anemia, or to low flow (i.e., inadequate perfusion), or both, is responsible for postoperative complications and increased post-operative surgical mortality, often due to multi-organ failure including renal and gastrointestinal organ systems. For a review of these papers, one may consult U.S. Patent Application Publication No. US 2006/0257283 A1. While certain patient parameter's may be measured during cardiopulmonary bypass, such as hematocrit % (HCT), hemoglobin (g/dL), arterial oxygen saturation (%), arterial oxygen tension (mm Hg), etc., despite intraoperative measures taken to maintain adequate oxygen delivery based on these parameters, inadequate oxygen delivery and tissue hypoxia may still occur during cardiopulmonary bypass procedures as evident from postoperative elevated levels of blood lactate levels. See, e.g., Marco Ranucci et al., Anaerobic Metabolism During Cardiopulmonary Bypass: Predictive Value of Carbon Dioxide Derived Parameters. 81 ANNUALS THORACIC SURGERY 2189-95 (2006).

Elevated post-operative blood lactate levels may reflect tissue hypoxia (type A hyperlactatemia) occurring during cardiopulmonary bypass rather than hyperlactatemia unrelated to hypoxia (type B hyperlactatemia). However, blood lactate levels do not provide an adequate monitoring parameter for tissue hypoxia during cardiac pulmonary bypass (whether due to hypoperfusion and/or inadequate blood oxygenation). Elevated blood lactate levels are a late indicator of tissue hypoxia that take hours to normalize. Consequently, monitoring blood lactate level during cardiopulmonary bypass procedures may not help clinicians avoid postoperative complications from intraoperative tissue hypoxia because elevated blood lactate levels mainly identify when tissue hypoxia has already occurred. Subsequent episodes of tissue hypoxia are difficult to recognize once hyperlactatemia is present. Thus, monitoring blood lactate levels may not help prevent irreversible tissue damage during cardiopulmonary bypass procedures.

Recently, it has been postulated that the best predictor of elevated lactate levels resulting from tissue hypoxia is the ratio of indexed oxygen delivery (DO2i) to indexed carbon dioxide elimination (VCO2i). Rannuci et al., Anaerobic Metabolism During Cardiopulmonary Bypass: Predictive Value of Carbon Dioxide Derived Parameters. 81 ANNUALS THORACIC SURGERY 2193 (2006). However, this ratio is a complicated calculation derived from a number of measured variables, so its use has been limited to a retrospective clinical outcomes research. Furthermore, there are no systems available that would allow a user to monitor such a complicated clinical predictor and, at the same time, facilitate through in-situ simulation in the operating room optimization of one or more complicated clinical predictors to improve patient care.

Reference US5,931,160 shows a ventilator control system controls a ventilator pneumatic system in a medical ventilator having a user interface. The user interface receives input values from a user for setting one or more breath parameters within a set of breath parameters. A processor simultaneously adjusts a plurality of controls within a ventilator pneumatic system in response to the set of breath parameters. The user can change or implement new phases, breaths, modes or therapies in seconds such that the therapy delivered to the patient is essentially uninterrupted.

US 2015/0105642 concerns a perfusion system for a CBP. The system comprises an oxygenator, several sensors, a processor or data management system DMS, data input facilities like keyboard and display for monitoring the patient's parameter during the procedure. The document also refers to several algorithms, in particular the Ranucci algorithm that is used to provide continuous and real-time information regarding the patient's oxygen delivery (DO2)and carbon dioxide production (VCO2).

Finally, US2014/099617 shows a medical simulation system (100) with a patient module. The patient module includes hydraulic equipment that simulates a baseline fluid interconnection with a therapeutic device. The computing device manages physical and virtual data, provides algorithmic calculations for simulating hypothetical patient vital signs, long-term clinical course, and simulates related fluid properties. The simulation system automatically executes a step-wise clinical scenario, specified in a spreadsheet format of patient conditions and equipment scenarios, that also includes audio/visual stimuli of operating room and diagnostic clinic environments, along with data recording capabilities.

There is a need in the field of cardiopulmonary bypass operations, and other similar operations and/or medical-surgical intensive care settings, for a system and method that can be used to calculate, simulate, and monitor complicated calculated indicators of adequate oxygen delivery and oxygen consumption in real time to prospectively make better clinical decisions. More generally, there is a need in the field of medicine and surgery for a system and method that can be used to calculate, simulate, and monitor complicated indicators of patient outcome and survival, namely, clinically relevant parameters for a patient, in real time, wherein each of these clinical parameters constitute complicated functions of multiple data input parameters and are useful in helping clinicians prospectively make better clinical decisions.

### SUMMARY OF THE DISCLOSURE

In accordance with this disclosure, an extracorporeal circulation system (10) comprising an oxygen delivery and consumption calculation-simulation-monitoring system (12) according to claim 1 is proposed. In a non-limiting embodiment of an oxygen delivery and consumption calculation-simulation-monitoring system operable to perform one or more functions directed to calculating, simulating and monitoring oxygen delivery, or oxygen consumption, or oxygen delivery and oxygen consumption for a patient the system includes among other aspects: an interface configured to receive data input pertaining to one or more input parameters selected from the group consisting of patient input parameters, perfusion input parameters, oxygen delivery input parameters, oxygen consumption input parameters, and carbon dioxide production input parameters; a processor operably connected to receive data signals from the interface corresponding to the received data input pertaining to the one or more Input parameters, wherein the processor calculates one or more output values based on the one or more input parameters, wherein the one or more output values are selected from the group consisting of at least one patient morphology value, at least one vascular fluids value, at least one oxygen delivery value, at least one oxygen consumption value and at least one carbon dioxide production value; and a monitor display assembly operably connected to receive the one or more output values calculated by the processor, wherein the monitor display assembly includes a display configured for monitoring at least one of the one or more output values calculated by the processor. In accordance with a second non-limiting embodiment of the first non-limiting system embodiment, the patient input parameters include a patient's height md the patient's weight, and the at least one patient morphology value includes a body surface area value for the patient.

In accordance with a third non-limiting embodiment of this system, the first and second non-limiting embodiments are modified so that the perfusion input parameters include a patient's pre-cardiac bypass blood volume per unit weight, the patient's pre-cardiac bypass hematocrit, an extracorporeal circuit priming volume, and a prime-off volume. In accordance with a fourth non-limiting embodiment of this system, the first, second and third non-limiting embodiments are modified so that the perfusion input parameters further include a hematocrit of packed red blood cells, a volume of a packed red blood cell infusion (RBC volume), and a volume of a fresh frozen plasma infusion and/or a volume expander infusion, and the at least one vascular fluids value includes an intra-cardiac bypass hematocrit value or an intra-cardiac bypass hemoglobin value. In accordance with a fifth non-limiting embodiment of this system, the first, second, third and fourth non-limiting embodiments are modified so that the oxygen delivery input parameters include a SaO₂ and a PaO₂, and the oxygen consumption input parameters include an SvO₂ and a PvO₂. In accordance with a sixth non-limiting embodiment of this system, the first, second, third, fourth and fifth non-limiting embodiments are further modified so that at least one oxygen delivery value is a function of cardiac bypass pump flow, the SaO₂ and the PaO₂, and at least one oxygen consumption value is a function of the cardiac bypass pump flow, the SvO₂, and the PvO₂, and the processor also calculates a ratio of oxygen delivery (DO₂) to oxygen consumption (VO₂), or a ratio of indexed oxygen delivery (DO₂ᵢ) to indexed oxygen consumption (VO₂ᵢ), or both ratios. In accordance with a seventh non-limiting embodiment of this system, the first, second, third, fourth, fifth and sixth non-limiting embodiments are modified so that the carbon dioxide production input parameters include an expired CO₂ and a gas flow (Qs) thru the oxygenator of a heart-lung machine. In accordance with an eighth non-limiting embodiment of this system, the first, second, third, fourth, fifth, sixth and seventh non-limiting embodiments are further modified so that at least one carbon dioxide production value is a function of the expired CO₂ and the gas flow (Qs) thru the oxygenator of a heart-lung machine, and the processor also calculates a ratio of oxygen delivery (DO₂) to carbon dioxide production (VCO₂), or a ratio of indexed oxygen delivery (DO₂ᵢ) to indexed carbon dioxide production (VCO₂ᵢ), or both the ratio of oxygen delivery (DO₂) to carbon dioxide production (VCO₂) and the ratio of indexed oxygen delivery (DO₂) to indexed carbon dioxide production (VCO₂ᵢ).

In accordance with a ninth non-limiting embodiment of this system, the first, second, third, fourth, fifth, sixth, seventh, and eighth non-limiting embodiments are further modified so that the interface includes a manual input portion configured for manual input of at least one of the one or more input parameters by a user and a machine input portion configured to receive sensor-derived input from one or more sensors. In accordance with a tenth non-limiting embodiment of this system, the first, second, third, fourth, fifth, sixth, seventh, eighth and ninth non-limiting embodiments are further modified so that the manual input portion is configured for manual input of a plurality of input parameters by the user, wherein some of the input parameters are real data inputs and some of the input parameters are hypothetical data inputs. In accordance with an eleventh non-limiting embodiment of this system, the first, second, third, fourth, fifth, sixth, seventh, eighth, ninth and tenth non-limiting embodiments are further modified so that the one or more output values calculated by the processor include at least one real output value and at least one predicted outcome output value corresponding to a clinical simulation, wherein both the at least one real output value and the at least one predicted outcome output value are displayed on the display.

In accordance with a twelfth non-limiting embodiment of this system, the first, second, third, fourth, fifth, sixth, seventh, eighth, ninth, tenth and eleventh non-limiting embodiments are modified to further include a plurality of sensors operably connected to input data to the machine interface portion of the interface. In accordance with a thirteenth non-limiting embodiment of this system, the first, second, third, fourth, fifth, sixth, seventh, eighth, ninth, tenth, eleventh and twelfth non-limiting embodiments are further modified so that the plurality of sensors include two or more sensors independently selected from the group consisting of a CO₂ sensor disposed to measure carbon dioxide exhausted from an oxygenator, a venous blood sensor disposed to measure oxygen tension of mixed venous blood from a patient, an arterial blood sensor disposed to measure oxygen tension of arterial blood from the oxygenator, and a gas flow meter disposed to measure flow rate of a ventilation gas. In accordance with a fourteenth non-limiting embodiment of this system, the first, second, third, fourth, fifth, sixth, seventh, eighth, ninth, tenth, eleventh, twelfth and thirteenth non-limiting embodiments are modified so that the plurality of sensors further include a hematocrit sensor, or a hemoglobin sensor, disposed on an extracorporeal blood flow circuit connected to a patient. In accordance with a fifteenth non-limiting embodiment of this disclosure, the first, second, third, fourth, fifth, sixth, seventh, eighth, ninth, tenth, eleventh, twelfth, thirteenth and fourteenth non-limiting embodiments are modified so that a plurality of sensors are operably connected to input data to the machine interface portion of the interface, wherein the interface further comprises a snapshot mechanism, wherein when activated the snapshot mechanism autopopulates values of multiple input parameters received by the interface into a simulator portal, wherein the simulator portal permits manual modification of at least one of the autopopulated values so as to provide hypothetical data input to the processor prior to the calculation by the processor of the at least one predicted outcome output value corresponding to the clinical simulation

In accordance with a sixteenth non-limiting embodiment of this disclosure, a calculation-simulation-monitoring method for calculating, simulating and/or monitoring oxygen delivery, or oxygen consumption, or oxygen delivery and oxygen consumption, for a patient is provided, wherein the method includes the steps of: (a) inputting data via an interface configured to receive data input, wherein the inputted data pertains to one or more input parameters selected from the group consisting of patient input parameters, perfusion input parameters, oxygen delivery input parameters, oxygen consumption input parameters, and carbon dioxide production input parameters; (b) calculating one or more output values based on the one or more input parameters using a processor operably connected to receive data signals from the interface corresponding to the one or more input parameters, wherein the one or more output values are selected from the group consisting of one or more patient morphology values, one or more vascular fluids values, one or more oxygen delivery values, one or more oxygen consumption values and one or more carbon dioxide production values; and (c) displaying the one or more output values calculated by the processor using a monitor display assembly, wherein the monitor display assembly includes a display configured for monitoring at least one of the one or more output values calculated by the processor. In accordance with a seventeenth non-limiting embodiment of this disclosure, the sixteenth non-limiting embodiment is modified so that some data inputted via the interface is manually inputted via a manual interface portion of the interface and some data inputted via the interface is sensor-derived data inputted via a machine interface portion of the interface. In accordance with an eighteenth non-limiting embodiment of this disclosure, the sixteenth and seventeenth non-limiting embodiments are modified so that the manual input portion is configured for manual input of a plurality of input parameters by the user, wherein some of the input parameters are real data inputs and some of the input parameters are hypothetical data inputs. In accordance with an nineteenth non-limiting embodiment of this disclosure, the sixteenth, seventeenth and eighteenth non-limiting embodiments are further modified so that the one or more output values calculated by the processor include at least one real output value and at least one predicted outcome output value that are both displayed on the display, wherein the at least one predicted outcome output value corresponds to a clinical simulation performed in real time as part of patient care. In accordance with a twentieth non-limiting embodiment of this disclosure, the sixteenth, seventeenth, eighteenth and nineteenth non-limiting embodiments are further modified so that a plurality of sensors are operably connected to input data to the machine interface portion of the interface, and so that the interface further comprises a snapshot mechanism, and the method further includes the steps of: activating the snapshot mechanism to autopopulate values of multiple input parameters received by the interface into a simulator portal; and modifying at least one of the autopopulated values so as to provide hypothetical data input to the processor, then employing the processor to calculate the at least one predicted outcome output value corresponding to the clinical simulation.

In accordance with a twenty-first non-limiting embodiment of this disclosure, an oxygen delivery and consumption calculation and monitoring system operable to continuously calculate and display one or more clinically relevant outcome parameters pertaining to oxygen delivery, or oxygen consumption, or oxygen delivery and oxygen consumption, for a patient is provided, wherein the system includes: an interface configured to receive data input pertaining to a plurality of input parameters from a plurality of sensors, wherein the plurality of input parameters include one or more perfusion input parameters, one or more oxygen delivery input parameters, one or more oxygen consumption input parameters, and one or more carbon dioxide production input parameters; a processor operably connected to receive data signals from the interface corresponding to the received data input pertaining to the plurality of input parameters, wherein the processor calculates one or more output values based on the plurality of input parameters, wherein the one or more output values are selected from the group consisting of at least one oxygen delivery value, at least one oxygen consumption value and at least one carbon dioxide production value; and a monitor display assembly operably connected to receive the one or more output values calculated by the processor, wherein the monitor display assembly includes a display configured to permit continuous monitoring of at least one of the one or more output values calculated by the processor, wherein the one or more output values calculated by the processor and continuously monitorable on the display include at least a ratio of a calculated oxygen delivery value to a calculated carbon dioxide production value (DO2/VCO2 or DO2i/VCO2i). In accordance with a twenty-second non-limiting embodiment of this disclosure, the twenty-first non-limiting embodiment is modified so that the display of the monitor display assembly includes a liquid crystal display controlled by the processor to display normal values of each calculated output value in a first color, and to display abnormal values within a first deviant range in a second color that is substantially different from the first color, and to display abnormal values within a second deviant range in a third color that is substantially different from the first color and the second color.

In accordance with a twenty-third non-limiting embodiment of this disclosure, a clinical parameter calculation-simulation-monitoring system is provided that is operable to perform one or more functions directed to calculating, simulating and monitoring one or more clinical parameters for a patient, wherein each of these clinical parameters are functions of multiple data input parameters, wherein the system includes: an interface configured to receive data input pertaining to one or more input parameters; a processor operably connected to receive data signals from the interface corresponding to the received data input pertaining to the one or more input parameters, wherein the processor calculates one or more output values based on the one or more input parameters, wherein the one or more output values pertain to clinically relevant outcome parameters; and a monitor display assembly operably connected to receive the one or more output values calculated by the processor, wherein the monitor display assembly includes a display configured for monitoring at least one of the one or more output values calculated by the processor. In accordance with a twenty-fourth non-limiting embodiment of this disclosure, the twenty-third non-limiting embodiment is modified so that some of the input parameters are real data inputs and some of the input parameters are hypothetical data inputs, and the one or more output values calculated by the processor include at least one predicted outcome output value corresponding to a clinical simulation, wherein the at least one predicted outcome output value is displayed on the display. In accordance with a twenty-fifth non-limiting embodiment of this disclosure, the twenty-third and twenty-fourth non-limiting embodiments are further modified so that the processor also calculates at least one real output value that is also displayed on the display with the at least one predicted outcome output value so that the at least one real output value may be monitored, and compared with the at least one predicted outcome value. In accordance with a twenty-sixth non-limiting embodiment of this disclosure, the twenty-third, twenty-fourth and twenty-fifth non-limiting embodiments are further modified so that the interface includes a manual interface portion and a machine interface portion, and some data inputted via the interface is manually inputted via the manual interface portion and some data inputted via the interface is sensor-derived data inputted from one or more sensors via the machine interface portion. In accordance with a twenty-seventh non-limiting embodiment of this disclosure, the twenty-third, twenty-fourth, twenty-fifth and twenty-sixth non-limiting embodiments are modified so that the interface includes a manual interface portion and a machine interface portion, and some data inputted via the interface is manually inputted via the manual interface portion and some data inputted via the interface is sensor-derived data inputted from one or more sensors via the machine interface portion, and the interface further comprises a snapshot mechanism, wherein when activated the snapshot mechanism autopopulates values of multiple input parameters received by the interface into a simulator portal, wherein the simulator portal permits manual modification of at least one of the autopopulated values so as to provide hypothetical data input to the processor prior to the calculation by the processor of the at least one predicted outcome output value corresponding to the clinical simulation.

In accordance with a twenty-eighth non-limiting embodiment of this disclosure, a perfusion calculation-simulation-monitoring system is provided that is operable to perform one or more functions directed to calculating, simulating and monitoring perfusion parameters for a patient, including hemoglobin, or hematocrit, or hemoglobin and hematocrit, wherein the system includes: an interface configured to receive data input pertaining to one or more input parameters selected from the group consisting of patient input parameters and perfusion input parameters; a processor operably connected to receive data signals from the interface corresponding to the received data input pertaining to the one or more input parameters, wherein the processor calculates one or more output values based on the one or more input parameters, wherein the one or more output values are selected from the group consisting of at least one patient morphology value and at least one vascular fluids value; and a monitor display assembly operably connected to receive the one or more output values calculated by the processor, wherein the monitor display assembly includes a display configured for monitoring at least one of the one or more output values calculated by the processor. In accordance with a twenty-ninth non-limiting embodiment of this disclosure, the twenty-eighth non-limiting embodiment is modified so that some of the input parameters are real data inputs and some of the input parameters are hypothetical data inputs, and the one or more output values calculated by the processor include at least one predicted outcome output value corresponding to a clinical simulation, wherein the at least one predicted outcome output value is displayed on the display. In accordance with a thirtieth non-limiting embodiment of this disclosure, the twenty-eighth and twenty-ninth non-limiting embodiments are further modified so that the processor also calculates at least one real output value that is also displayed on the display with the at least one predicted outcome output value so that the at least one real output value may be monitored, and compared with the at least one predicted outcome value. In accordance with a thirty-first non-limiting embodiment of this disclosure, the twenty-eighth, twenty-ninth and thirtieth non-limiting embodiments are further modified so that the at least one predicted outcome output value is a patient's intra-cardiopulmonary bypass hemoglobin or hematocrit. In accordance with a thirty-second non-limiting embodiment of this disclosure, the twenty-eighth, twenty-ninth, thirtieth and thirty-first non-limiting embodiments are modified so that the interface includes a manual interface portion and a machine interface portion, and some data inputted via the interface is manually inputted via the manual interface portion and some data inputted via the interface is sensor-derived data inputted from one or more sensors via the machine interface portion, and the interface further comprises a snapshot mechanism, wherein when activated the snapshot mechanism autopopulates values of multiple input parameters received by the interface into a simulator portal, wherein the simulator portal permits manual modification of at least one of the autopopulated values so as to provide hypothetical data input to the processor prior to the calculation by the processor of the at least one predicted outcome output value corresponding to the clinical simulation.

### Brief Descriptions of the Drawings

Figure 1A is a prospective view of a cardiopulmonary bypass system provided with an oxygen delivery and consumption calculation-simulation-monitoring system; and Figure 1B is a schematic drawing of a cardiopulmonary bypass system provided with an oxygen delivery and consumption calculation-simulation-monitoring system in accordance with an embodiment of this disclosure.
Figure 2 is a schematic drawing of an oxygen delivery and consumption calculation-simulation-monitoring system according to an embodiment of this disclosure.
Figure 3 is a schematic data flow diagram illustrating input parameters inputted into a processor comprising various calculations circuits, and the outputs of these circuits, in accordance with an embodiment of this disclosure.
Figure 4 is a non-limiting configuration of a graphical user interface (GUI) display for monitoring patient clinical parameters, and comparing hypothetical actions and outcomes with the results of actual actions and outcomes in accordance with an embodiment of this disclosure.
Figure 5 is an interaction flow diagram showing steps of a method for simulating oxygen delivery and oxygen consumption for a patient in accordance with an embodiment of this disclosure.
Figure 6A is a schematic drawing of an oxygen delivery and consumption monitoring system according to an embodiment of this disclosure, and Figure 6B is a graphical user interface corresponding to the oxygen delivery and consumption monitoring system according to the embodiment of Figure 6A.

### Detailed Description of Embodiments of this Disclosure

Various embodiments in accordance with this disclosure are described with reference to the figures, in which like parts are designated by like reference numbers. The drawings described herein constitute non-limiting illustrations.

As shown in FIGs. 1A and 1B, an extracorporeal circulation system 10 (also referred to as a heart-lung machine, or as a cardiac bypass system, or as a cardiopulmonary bypass system, and which should be construed broadly to include cardiopulmonary bypass (CPB) systems, minimal extracorporeal circulation (MECC) systems, extracorporeal membrane oxygenation (ECMO) systems (respiratory and cardiac), and pump assisted lung protection (PALP) systems) includes an oxygen delivery and consumption calculation-simulation-monitoring system 12, as well as a venous reservoir 14, a blood pump 16 and an oxygenator 18. The venous reservoir 14, blood pump 16 and oxygenator 18 are connected together in a conventional manner so that venous blood extracted from a cavoatrial cannula 20 inserted in the venous side of a patient's circulatory system, for example the vena cava and/or the right atrium of the heart H, flows via tubing 22 into the venous reservoir 14, and from there is pumped via the blood pump 16 into the oxygenator 18, which oxygenates the blood before the blood, now oxygenated, flows through tubing 24 and into arterial cannula 26 inserted in the aortic root of the heart H. The blood pump 16 may be a roller pump or a centrifugal pump.

A venous blood sensor 28 may be connected to the tubing 22 to provide sensor input (i.e., SvO₂, PvO₂) to the calculation-simulation-monitoring system 12. An arterial blood sensor 29 may be connected to tubing 24 to provide sensor input (i.e., SaO₂, PaO₂) to the calculation-simulation-monitoring system 12. The blood sensors 28, 29 may each be constructed as a single sensor, or as multiple sensors, so long as they measure blood oxygenation parameters SaO₂, PaO₂, etc., pertaining to the amount of oxygen carried by the blood. A flowmeter 30 may be provided between the blood pump 16 and the oxygenator 18 to measure the flow rate of blood exiting the blood pump 16, and to input blood flow rate data Qp to the calculation-simulation-monitoring system 12. If the blood pump 16 is a roller pump, then the RPM-based flow rate measuring system of the roller pump may input flow rate data to the calculation-simulation-monitoring system 12, and the flowmeter 30 may be omitted, or both the RPM-based flow rate measuring system and the flowmeter 30 may both input flow rate data to the calculation-simulation-monitoring system 12 to provide a dual flow rate measurement capability.

A CO₂ sensor or capnograph 32 may be connected to the gas escape of oxygenator 18 so that exhaled carbon dioxide (expCO₂) data may be continuously measured and input into the calculation-simulation-monitoring system 12. Various suitable CO₂ sensors 32 are commercially available and may be employed to provide exhaled carbon dioxide (expCO₂) data to the system 12. Such suitable CO₂ sensors 32 may be reusable.

A hematocrit (HCT) sensor 34 may be placed on the venous or arterial side of the blood flow circuit through tubes 22 and 24. For example, the HCT sensor 34 may be placed on the venous side between either the reservoir 14 and the blood pump 16, or between the blood pump 16 and the oxygenator 18, or the HCT sensor 34 may be placed on the arterial side downstream of the oxygenator 18. In this context, the venous side of the blood flow circuit refers to deoxygenated blood flowing from the cavoatrial cannula 20, the reservoir 14, the blood pump 16 and the tubing 22, and the arterial side of the blood flow circuit refers to oxygenated blood flowing from the oxygenator 18, the tubing 24 and the arterial catheter 26. The HCT sensor 34 is operably connected to input HCT data into the calculation-simulation-monitoring system 12. Any suitable, commercially available HCT sensor may be used, such as CRIT-LINE® (HemaMetrics, Kaysville, UT). Such HCT sensors 34 are preferably disposable.

In the alternative, the HCT sensor 34 may be replaced with a non-invasive hemoglobin (Hb) sensor, such as the Masimo Radical-7 Pulse CO-Oximeter (Masimo Corporation, Irvine, CA), modified to input Hb data into the calculation-simulation-monitoring system 12. Of course, the use of both the HCT sensor 34 and a Hb sensor falls within the scope of this disclosure so that both HCT data and Hb data are continuously input to the calculation-simulation-monitoring system 12.

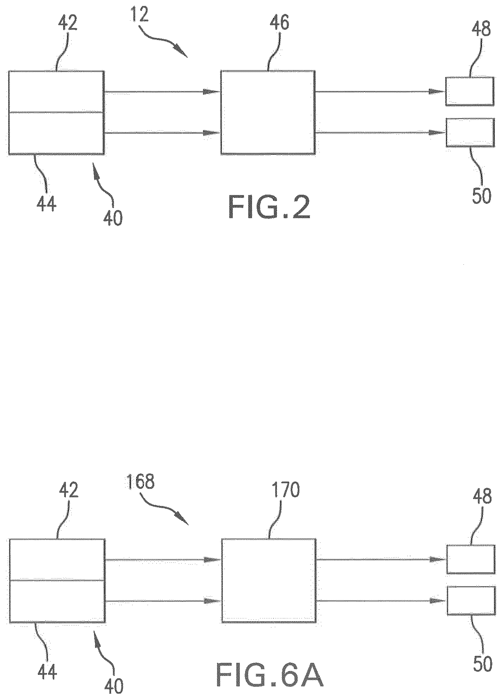

With reference to FIG. 2, the oxygen delivery and consumption calculation-simulation-monitoring system 12 includes an interface 40 comprising a manual interface portion 42 and a machine interface portion 44. The interface 40 is operably connected to provide data input to a processor 46, which employs the data in calculations of output values indicative of the patient's condition, or of output values simulating outcomes of hypothetical or planned clinical intervention. The processor 46 is connected to a display assembly 48, which is used to display output values calculated by the processor 46, and to a memory assembly 50 that is used to store output values and non-outputted values calculated by the processor 46. The memory assembly 50 may include both RAM and ROM components, and/or other devices suitable for data storage. In accordance with an embodiment of this disclosure, processor 46 is not a general computer. On the contrary, processor 46 may be an embedded system with particular dedicated functions (i.e., such as those described herein) within the larger electrical system of the calculation-simulation-monitoring system 12.

The manual interface portion 42 is configured so that a user may manually input data into the calculation-simulation-monitoring system 12. For example, certain data pertaining to a patient are substantially static data with respect to the cardiopulmonary bypass procedure to be performed. Examples of such static patient data includes patient morphological data such as height and weight, which will not substantially change during the cardiopulmonary bypass procedure. Such static data pertaining to the patient at the start of the cardiopulmonary bypass procedure may be referred to as patient input parameters. Thus, in accordance with this disclosure, static data constitutes data collected before, or at the beginning of, a medical and/or surgical procedure and constitutes data that does not change appreciably during the course of the medical and/or surgical procedure.

Other static data that a user may input via the manual interface portion 42 of the interface 40 include perfusion input parameters such as the patient's pre-cardiac bypass blood volume per unit weight, and the patient's pre-cardiac bypass hematocrit. These perfusion input parameters pertain to the patient's starting condition at the beginning of the cardiopulmonary bypass procedure. While hematocrit, for instance, may change during the cardiopulmonary bypass procedure, its starting value at the beginning of the procedure does not. Consequently, it constitutes data that a user may input manually into the system 12 via the manual interface portion 42.

Other perfusion input parameters that may be manually input via the manual interface portion 42 include an extracorporeal priming volume and a prime-off volume. The priming volume constitutes the volume of fluid used to prime the tubing of the cardiopulmonary bypass system 10, such as tubes 22 and 24, and other tubes generally included in sub-circuits of the system pertaining to various safety features, etc. The priming volume is static data in that it is fixed at the beginning of the cardiopulmonary bypass procedure, and is a parameter that does not change once the cardiopulmonary bypass procedure begins. The prime-off volume constitutes that volume of priming fluid that is recovered from the extracorporeal circuit prior to the onset of the cardiopulmonary bypass procedure, and is another parameter set at the beginning of the procedure that does not change later during the procedure.

Another perfusion input parameter that may be manually input via the manual interface portion 42 includes the institutional hematocrit (HCT) of packed red blood cells. As is known in the art, the HCT of packed red blood cells used by various hospitals and like institutions for patient transfusion vary from institution to institution. At the beginning of a cardiopulmonary bypass procedure, a user may manually enter this data with respect to the HCT of packed red blood cells at the particular institution via the manual interface portion 42 of the interface 40 for use as data input for the calculation-simulation-monitoring system 12.

The user may also be able to enter dynamic data for the calculation-simulation-monitoring system 12 using the manual interface portion 42. Dynamic data pertains to variables that change during the cardiopulmonary bypass procedure. Examples of dynamic data variables include the volume of packed red blood cells transfused, and other input fluids infused, into the patient during the cardiopulmonary bypass procedure, the pump flow Qp (1/min), arterial oxygen tension PaO₂ (mm Hg), mixed venous oxygen saturation SvO₂ (%), mixed venous oxygen tension PvO₂ (mm Hg), exhaled carbon dioxide expCO₂ (mm Hg), and ventilation Qs (1/min). Actual PvO₂ data may be measured inline or by means of discrete blood gas sampling. Preferably, data inputs pertaining to such dynamic data variables, which may be directly measured in a continuous fashion and/or calculated from one or more variables directly measured in a continuous fashion, are input into the machine interface portion 44 from sensors and/or processors responsible for generating the dynamic data. However, the manual interface portion 42 permits the user to supplement this otherwise automatic flow of data via the machine interface portion 44 with data collected from a secondary source (e.g., hospital lab) for the purpose of correlating with data collected by the sensors and processors connected to the calculation-simulation-monitoring system 12. In addition, providing the user with the ability to manually enter values for the dynamic data permits the user to enter hypothetical values, and/or estimated values, and/or to enter guesstimated values, for the dynamic data in order to perform real-time simulations and/or to test "what if" scenarios during the cardiopulmonary bypass procedure, or during other surgical procedures, or during other critical care situations involving cardiopulmonary bypass technology.

The machine interface portion 44 of the interface 40 is operably connected to receive data from various sensors and/or processors that generate data used to calculate, simulate and/or monitor values of predictive indicators of the patient's condition during the cardiopulmonary bypass procedure. For example, as evident from FIGs. 1B and 2, the venous blood sensor 28 is operably connected to provide continuous data input to the machine interface portion 44 for mixed venous oxygen tension PvO₂ data and/or mixed venous oxygen saturation SvO₂ data. The HCT sensor 34 is operably connected to provide continuous HCT data input to the machine interface portion 44. Optionally as an addition to, or as an alternative to, the HCT sensor 34, a Hb sensor may be operably connected to provide continuous Hb data input to the machine interface portion 44. When the blood pump is a centrifugal pump, then the flowmeter 30 is provided and is operably connected to provide continuous pump flow Qp data input to the machine interface portion 44. When the blood pump is a roller pump, then the flowmeter 30 may still be used in the same manner as when the blood pump is a centrifugal pump, or the flowmeter 30 may be omitted and pump flow Qp data may be continuously input to the machine interface portion 44 from an RPM-based flow rate measuring system that is integral to the roller pump assembly, or via an external flow probe.

As evident from FIGs. 1B and 2, the CO₂ sensor 32 may be operably connected to the machine interface portion 44 so as to provide continuous expCO₂ data input. The various sensors 28, 32 and 34, and flowmeter 30, are merely non-limiting examples of sensors and like devices that may be operably connected to the machine interface portion 44 in order to provide continuous data input to the calculation-simulation-monitoring system 12. For example, the extracorporeal circulation system 10 could be provided with an in-line sensor (not shown) that provides data input to the processor 46 for comparison purposes, and so that blood lactate values are continuously monitored and displayed by display assembly 48.

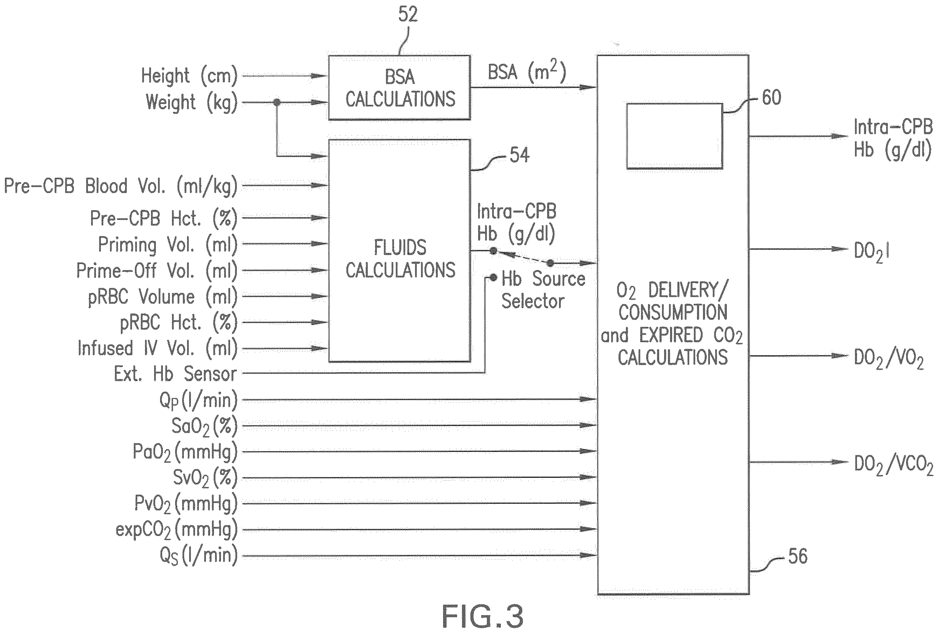

FIG. 3 illustrates various components of the processor 46 of the calculation-simulation-monitoring system 12, which performs the various calculations and hypothetical simulations as are described below. Processor 46 includes a BSA calculation circuit 52 ("BSA circuit"), a fluids calculations circuit 54 ("fluids circuit"), and an oxygen delivery/oxygen consumption and expired CO₂ calculations circuit 56 ("oxygen/CO₂ circuit"). In this context, each of these circuits may constitute physically separate circuits, or they may share and/or overlap their circuitry components, relying on programming code to distinguish the circuits 52, 54 and 56 from one another, in whole or in part.

The BSA circuit 52 receives a patient's height and weight data input from the manual interface portion 42, and then operates to calculate the patient's Body Surface Area (BSA) based on this data. The BSA is calculated using one of the known equations for BSA, namely, either the DuBois & DuBois equation, or the Boyd equation, or the Mosteller equation. Preferably, the BSA circuit 52 is programmed to selectively carry out the calculation of BSA based on one of these equations selected by the user via the manual interface portion 42. The three equations are listed below as follows:
(1) DuBois and Dubois:$BSA \left({m}^{2}\right) = 0.007184 \times height {\left(cm\right)}^{0.725} \times weight {\left(kg\right)}^{0.425} ;$ (1)
(2) Boyd: $BSA \left({m}^{2}\right) = 0.0003207 \times height {\left(cm\right)}^{0.3} \times {\left[weight\left(kg\right)\times 1000\right]}^{0.7285 - 0.0188 log \left[weight\left(kg\right)\times 1000\right]} ;$ (2)
(3) Mosteller: $BSA \left({m}^{2}\right) = \left({\left[height\left(cm\right)\times weight\left(kg\right)\right]}^{1 / 2}\right) / 60 .$ (3)

While the BSA circuit 52 preferably employs the BSA equations described above, the BSA circuit 52 may employ any valid mathematical model for calculating BSA. The BSA circuit 52 outputs the calculated BSA to the oxygen/CO₂ circuit 56 for use in calculations by the oxygen/CO₂ circuit 56. The calculated BSA may also be outputted to the display assembly 48 for display.

The fluids circuit 54 receives multiple data inputs and is programmed to calculate intra-cardiopulmonary bypass (intra-CPB) Hb (g/dl) based on these multiple data inputs. The calculation performed by the fluids circuit 54 provides valuable information regarding how the patient's hemoglobin has changed, or should change, in view of fluids infused and blood products transfused into the patient during cardiopulmonary bypass, or in view of fluids and blood products contemplated to be infused and transfused, respectively, into the patient during cardiopulmonary bypass.

More specifically, the fluids circuit 54 receives data inputted from the manual user interface 42, such as the patient's weight and the patient's pre-cardiopulmonary bypass (pre-CPB) blood volume (ml) per unit weight (kg). The patient's pre-CPB blood volume is itself a calculated value that may be determined using the following formula: $Pre-CPB Blood Volume \left(ml\right) = \left[Blood volume \left(ml\right)/unit weight \left(kg\right)\right] \times weight \left(kg\right) .$ (4) The blood volume/unit weight has units of (ml/kg) and is an assumed ratio of blood volume to body weight for a particular patient. For example, a normal adult has a blood volume/unit weight ratio of 70 ml/kg, although this ratio may change with certain medical conditions such as congestive heart failure. See, e.g., Robert K. Funkhouser et al., Change in Relationship of Blood Volume to Weight in Congestive Heart Failure, 16 CIRCULATION 548-557 (1957). If the patient is a pediatric patient, a blood volume/unit weight ratio of 85 ml/kg may be employed in place of the blood volume/unit weight ratio employed for adults. While pre-CPB blood volume is preferably calculated based on an assumed ratio of blood volume to body weight, it is within the scope of this disclosure to employ other generally accepted calculations for estimating pre-CPB blood volume such as those methods employing both the patient's height and weight, and those methods employing the patient's BSA. Furthermore, it is within the scope of this disclosure to employ other blood volume/unit weight ratios other than 70 ml/kg for adults and 85 ml/kg for pediatric patients.

The patient's pre-cardiopulmonary bypass HCT (%) is a measured value that is input to the fluids circuit 54 via the manual interface portion 42. The patient's pre-CPB HCT constitutes the measured value of the patient's HCT prior to the start of the cardiopulmonary bypass procedure. Other data inputs pertinent to the fluid calculations to be calculated by the fluids circuit 54 include the priming volume (ml) and the prime-off volume (ml). The priming volume is the volume of fluid added to the extracorporeal blood circuit, such as tubes 22, 24, etc., of the cardiopulmonary bypass (CPB) system 10 in order to displace air from this new extension of the circulatory space. The priming volume is necessary to ensure, upon activation of the CPB system 10, rapid achievement of adequate blood flow rates without inducing air embolisms. The prime-off volume is the volume of the priming solution that is, in some cases, recovered from the extracorporeal blood circuit by a perfusionist before cardiopulmonary bypass is initiated with mixing of the patient's blood with the priming solution. Recovering priming solutions helps reduce the amount of hemodilution experienced by the patient during cardiopulmonary bypass. Because the priming volume and prime-off volume are generally determined by the perfusionist operating the CPB system 10, the priming volume and prime-off volume constitute static data that must be manually entered via the manual interface portion 42 for use by the fluids circuit 54.

The HCT of packed red blood cells (pRBCs) for the institution in which the cardiopulmonary bypass procedure is performed is manually entered via the manual interface portion 42 for use by the fluids circuit 54. The pRBC HCT constitutes static data that is determined by each institution providing pRBC transfusions.

Two dynamic data parameters that may need to be input by the user via the manual interface portion 42 during the CPB procedure are the volume (ml) of pRBCs transfused during the cardiopulmonary bypass procedure and the volume (ml) of other fluids infused into the patient during the cardiopulmonary bypass procedure. Examples of other fluids other than pRBCs that may be infused into the patient during the CPB procedure, and which may impact the calculated intra-CPB Hb and HCT result, include other blood products, such as fresh frozen plasma, and volume expanders, such as crystalloids including, but not limited to, Ringer's Lactate, normal saline (NS), D5W, D5W NS, D5W and 1/2NS, and etc.

In view of these inputs, the fluids circuit 54 calculates the intra-CPB Hb, and this calculated Hb value may be input into the oxygen/CO₂ circuit 56 for use in the calculations of that circuit. In addition, the calculated Hb value provided by the fluids circuit 54 may be outputted to and displayed by the display assembly 48. The fluids circuit 54 calculates the intra-CPB Hb from the intra-CPB HCT (%) using the relationship that intra-CPB Hb is one third the intra-CPB HCT. While this approximation is employed in an embodiment of the calculated Hb value, in accordance with this disclosure other valid relationships and/or tables for converting HCT values to Hb values may be used.

The intra-CPB HCT (%) is calculated by the following formula: $Intra-CPB HCT \left(%\right) = [ Intra-CPB RBC Cellular Volume \left(ml\right) / \left[Intra-CPB Blood Volume \left(ml\right)\right] \times 100 ,$ (5) wherein intra-CPB RBC Cellular Volume (ml) is the sum of pre-CPB RBC cellular volume (ml) and transfused RBC cellular volume (ml) and minus any substantial cellular blood loss during the CPB surgery. The pre-CPB RBC cellular volume is determined from the following formula: $Pre-CPB RBC cellular volume \left(ml\right) = \left[Pre-CPB Blood Volume \left(ml\right)\right] \times \left[Pre-CPB HCT\left(%\right)\right] .$ (6) The transfused RBC cellular volume (ml) is simply the product of the total volume of pRBCs that have been transfused into the patient during the cardiopulmonary bypass procedure, and up to that point in time, and the HCT (%) of the pRBCs.

In other words, the Transfused RBC cellular volume (ml) is represented by the following formula: $Transfused RBC cellular volume \left(ml\right) = \left[Total pRBC Volume Transfused \left(ml\right)\right] \times \left[pRBC HCT \left(%\right)\right] .$ (7) The blood loss cellular volume may be estimated by the product of [Total blood loss estimated (ml)] x [Pre-CPB HCT (%)]. Accounting for surgical blood losses during cardiopulmonary bypass surgery is optional in accordance with an embodiment of this disclosure. Furthermore, the Intra-CPB cellular volume may be determined by the following summation: $Intra-CPB Cellular Volume \left(ml\right) = Pre-CPB RBC Cellular Volume \left(ml\right) + Transfused RBC Cellular Volume \left(ml\right) - Blood Loss Cellular Volume \left(ml\right) .$ (8)

The Intra-CPB blood volume (ml) at any point in time during the cardiopulmonary bypass procedure is determined from the following formula: $Intra-CPB Blood Volume \left(ml\right) = Pre-CPB Blood Volume \left(ml\right) + Total pRBC Volume Transfused \left(ml\right) + Total Fluid Volume Infused \left(ml\right) + Priming Volume \left(ml\right) - Prime-off Volume \left(ml\right) - Estimated Surgical Blood Loss .$ (9) The volumes pertaining to the total pRBC volume transfused and the total fluid volume infused are total volumes determined up to the point in time during the cardiopulmonary bypass procedure, for which the Intra-CPB blood volume is calculated. Likewise, the estimated surgical blood loss volume is a volume determined up to the point in time during the cardiopulmonary bypass procedure, for which the Intra-CPB blood volume is calculated. In accordance with an embodiment of this disclosure, the Intra-CPB blood volume may be calculated without accounting for estimated surgical blood loss. As discussed above, the total fluid volume infused pertains to fluids other than pRBCs, such as fresh frozen plasma, and crystalloids including but not limited to Ringer's Lactate, normal saline (NS), D5W, D5W NS, D5W and 1/2NS.

Thus, from measured values and/or calculated values determined from measured values, the patient's intra-CPB HCT (%) may be calculated, as well as the patient's intra-CPB Hb (g/dl). These calculated results may be outputted by the processor 46 to the display assembly 48 for display and to the memory assembly 50 for storage and later use and/or referral. In accordance with an embodiment of this disclosure, the calculation-simulation-monitoring system may be limited to a subassembly of the calculation-simulation-monitoring system 12, namely, the BSA calculations circuit 52 and the fluids calculation circuit 54, which may be characterized as a perfusion calculator. The perfusion calculator may output calculated values that pertain to intra-CPB HCT, and/or intra-CPB Hb, and/or the patient's BSA, directly to the display assembly 48. Thus, in accordance with an embodiment of this disclosure, the perfusion calculator constitutes a stand-alone module without the oxygen/CO₂ circuit 56, and constitutes a perfusion calculation-simulation-monitoring system operable to perform one or more functions directed to calculating, simulating and monitoring perfusion parameters for a patient, including hemoglobin, or hematocrit, or hemoglobin and hematocrit.

In accordance with an embodiment of this disclosure, the perfusion calculator is integrated with the oxygen/CO₂ circuit 56 to form an oxygen delivery and consumption calculation-simulation-monitoring system 12 operable to perform one or more functions directed to calculating, simulating and monitoring oxygen delivery, or oxygen consumption, or oxygen delivery and oxygen consumption, for a patient In this embodiment, the fluids circuit 54 outputs the calculated value for intra-CPB Hb (g/dl) as input into the oxygen/CO₂ circuit 56 for use in additional calculations.

It should be appreciated that the calculation-simulation-monitoring system 12 has two sources of values of intra-CPB Hb (g/dl) input to the oxygen/CO₂ circuit 56, namely, the calculated value provided by the fluids circuit 54 and a value provided by and/or derived from the HCT sensor 34 or from a Hb sensor. In this context, the value provided by, and/or derived from the HCT sensor or the Hb sensor may be referred to as a sensor-derived value. In an embodiment, the calculation-simulation-monitoring system 12 may be provided with a Hb source selector switch, which is used to selectively switch input regarding intra-CPB Hb between the fluids calculations circuit 54 and the HCT sensor 34 (or Hb sensor). In another embodiment of the calculation-simulation-monitoring system 12, both the fluids calculations circuit 54 and the HCT sensor/Hb sensor 34 provide HCT and/or Hb data input into the oxygen/CO₂ circuit 56, and it is the oxygen/CO₂ circuit 56 that decides, based on user control input, which source of HCT and/or Hb data input is used in the calculations performed by the oxygen/CO₂ circuit 56.

The advantage of having these dual sources of intra-CPB Hb values is as follows. First, the sensor-derived Hb values may be used to provide continuous monitoring of the patient's hemoglobin and HCT values based more directly on measured values. Second, the calculated Hb values provided by the fluids circuit 54 pertain to predicted hemoglobin and HCT values based on actual and/or exploratory inputs and outputs. Consequently, the processor 46 may be provided with a statistical circuit 60 that compares and correlates the sensor-derived Hb and/or HCT values with those theoretical actual Hb and/or HCT values calculated by the fluids circuit 54. If the correlation falls below a predetermined threshold, thereby indicating poor correlation, then the processor 46 may output a warning that is displayed by the display assembly 48. In one embodiment, the display assembly 48 displays the sensor-derived Hb and/or HCT with the Hb and/or HCT values calculated by the fluids circuit 54 for visual comparison by a user of the system 12, such as a perfusionist, nurse or physician. In another embodiment, the statistical correlation value, such as a correlation coefficient r and/or a coefficient of determination r², is also displayed with the Hb and/or HCT values derived from the sensor 34 and from the fluids circuit 54, with or without a displayed visual warning when the correlation value falls below a pre-determined threshold. When the correlation value indicates poor correlation between the sensor-derived and fluids circuit-derived values of Hb and/or HCT, this may alert the health care team to an otherwise undetected problem such as third-spacing of fluids into the patient's tissues and/or occult blood loss that is causing the poor correlation.

Another benefit of having dual sources of values for intra-CPB Hb and/or HCT is the option to test planned interventions hypothetically to predict a best course of action prior to implementing a therapeutic intervention. For example, a perfusionist may employ the calculation-simulation-monitoring system 12 to calculate out the present predicted intra-CPB Hb and/or HCT based on data inputs for actual total pRBC transfusion volume and actual total fluid infusion volume up to a particular point in time during the cardiopulmonary bypass procedure. On the other hand, the perfusionist may enter data inputs representing a hypothetical total pRBC transfusion volume and/or a hypothetical total fluid volume to see what the predicted Hb and/or HCT values would be if a particular treatment intervention was instituted, such as a blood transfusion, a fluid bolus infusion, and/or a change in cardiac pump speed.

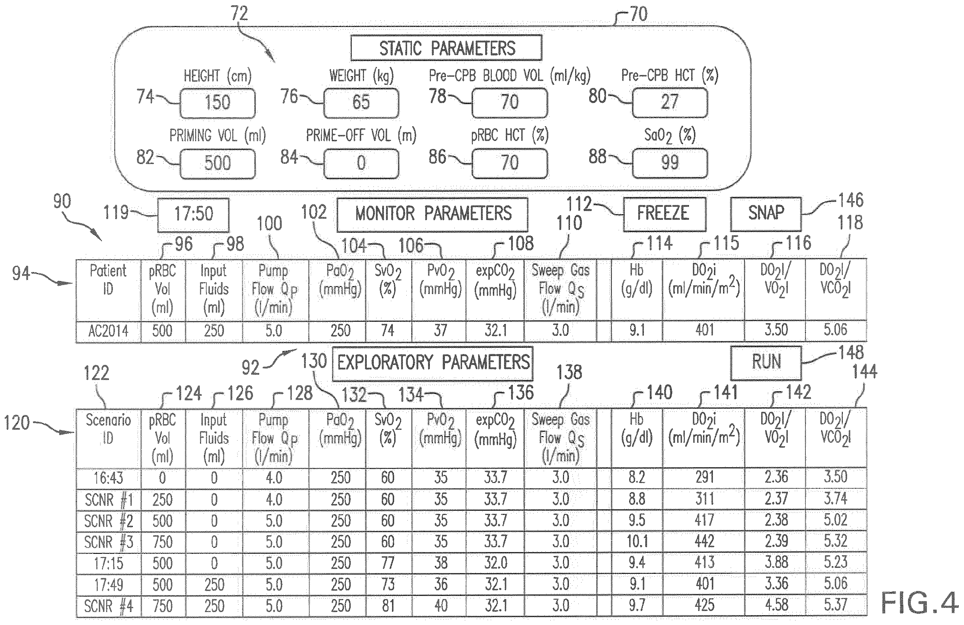

For example, at a time T during a cardiopulmonary bypass procedure (see, e.g., the illustrative example of FIG. 4, time T = 17:49), the patient may have received 500 ml pRBCs total transfusion and 250 ml of fluids, and has a calculated Hb of 9.1 and/or calculated HCT of 27.3, which correlates acceptably to the sensor-measured Hb of 9.1 and/or HCT of 27.3. The surgical team may be contemplating giving the patient another transfusion of pRBCs, and wishes to know the predicted change in Hb and/or HCT, and possibly also the outcome or impact on derived delivered oxygen (DO2) or indexed delivered oxygen (DO2i) at variable flow rates, depending upon whether one unit (250 ml) of pRBCs is transfused compared to transfusing the patient with two units (500 ml) of pRBCs. The user of the system 12 may use the manual interface portion 42 to enter the hypothetical data set including a hypothetical pRBC total transfusion volume of 750 ml and actual total fluid infusion volume of 250 ml so that the fluids circuit 54 may calculate out a first simulated Hb and/or HCT value (see, e.g., SCNR #4 of FIG. 4). The user of the system 12 may then use the manual interface portion 42 to enter another hypothetical data set including a hypothetical pRBC total transfusion volume of 1000 ml and actual total fluid infusion volume of 250 ml so that the fluids circuit 54 may calculate out a second simulated Hb and/or HCT value. These two simulated values are outputted by the fluids circuit 54 to the display assembly 48 so that the user may compare the calculated results in order to facilitate a clinical decision regarding which volume of pRBCs is predicted to achieve clinical goals while minimizing the risks of pRBC transfusion. The fluids circuit 54 may also output these two simulated values to the memory assembly 50 for storage and later consultation, and the data input entered via the manual interface portion 42 may likewise be stored by memory assembly 50 for later consultation.

The simulated calculations are not limited to pRBC transfusion. For example, during the cardiopulmonary bypass procedure, the surgical team may be contemplating an infusion of 1000 ml of ringer's lactate to maintain the blood pressure of the patient described above, who has already received 500 ml pRBC total transfusion and 250 ml of fluids. The user of the system 12 may use the manual interface portion 42 to enter hypothetical data corresponding to the planned infusion, i.e., a hypothetical total fluid infusion volume of 1250 ml and an actual total pRBC transfusion volume of 500 ml, which the fluids circuit 54 will use to calculate the corresponding predicted Hb and/or HCT as a simulation. If the surgical team decides that such an intervention would dilute the patient's Hb and/or HCT to an unacceptably low value (e.g., Hb below 8.3 g/dl and/or HCT below 25%), then the surgical team may consider intervening to give both a 250 ml transfusion of pRBC and 750 ml of fluids instead of the initially considered infusion of 1000 ml of ringer's lactate. In this case, the user of the system would use the manual interface portion 42 to enter the hypothetical data corresponding to the alternate intervention including both transfusion and infusion, so the entered values would include a hypothetical total infusion volume of 1000 ml and a hypothetical total pRBC transfusion volume of 750 ml. The fluids circuit 54 would then calculate out a simulated Hb and/or HCT value corresponding to this alternate planned clinical intervention.

In accordance with an embodiment of this disclosure, the display assembly 48 is configured to display at least two simulated Hb and/or HCT values with their corresponding data inputs. In accordance with another embodiment of this disclosure, the display assembly 48 is configured to display at least several simulated Hb and/or HCT values with their corresponding data inputs.

As shown in FIG. 3, the oxygen/CO₂ circuit 56 may receive the calculated intra-CPB Hb as input from the fluids circuit 54, and it may also receive the sensor-derived Hb and/or HCT measurement provided by the HCT sensor 34 or Hb sensor. In one embodiment, the oxygen/CO₂ circuit 56 includes the statistical circuit 60 that compares and correlates the sensor-derived Hb and/or HCT values with those Hb and/or HCT values calculated by the fluids circuit 54. In one embodiment, the oxygen/CO₂ circuit 56 employs only the sensor-derived Hb value in its further calculations with respect to calculated values pertaining to oxygen delivery and/or oxygen consumption and/or expired carbon dioxide. In one embodiment, the oxygen/CO₂ circuit 56 employs only the Hb value calculated by the fluids circuit 54 in its further calculations with respect to calculated values pertaining to oxygen delivery and/or oxygen consumption and/or expired carbon dioxide. In one embodiment, the oxygen/CO₂ circuit 56 employs both the sensor-derived Hb value and the Hb values calculated by the fluids circuit 54 in its further calculations with respect to one or more calculated values pertaining to oxygen delivery and/or oxygen consumption and/or expired carbon dioxide, wherein a Hb source selector switch, actuatable from the manual interface portion 42 depending on whether hypothetical Hb values or actual measured Hb values, is provided so that the source of Hb data input may be switched between calculated Hb data input from the fluids calculation circuit 54 and sensor-derived Hb data input from the HCT or Hb sensor 34.

The oxygen/CO₂ circuit 56 employs intra-CPB Hb, whether sensor-derived or calculated by the fluids circuit 54, to calculate values pertaining to oxygen delivery to the patient, oxygen consumption by the patient and/or carbon dioxide production by the patient. As shown in FIG. 3, the oxygen/CO₂ circuit 56 receives additional data input with respect to arterial pump flow Qp (L/min) of the blood pump 16, which is either provided by the flowmeter 30 when either a roller pump or a centrifugal pump is used, or by the RPM-based flow rate measuring system of the roller pump when a roller pump is used. The oxygen/CO₂ circuit 56 also receives input from venous blood sensor 28, which provides input data pertaining to mixed venous oxygen tension PvO₂ (mm Hg), which is a measure of the partial pressure of dissolved oxygen in venous blood returning to the heart-lung machine 10. The oxygen/CO₂ circuit 56 also receives input from CO₂ sensor or capnograph 32, which provides input data pertaining to expired/exhaled carbon dioxide (expCO₂) present in the exhaust gas from the oxygenator 18.

The oxygen/CO₂ circuit 56 is constructed and/or programmed to calculate oxygen delivery (DO2, ml/min) to the patient during cardiopulmonary bypass based on the following formula: $DO 2 \left(ml/min\right) = Qp \left(L/min\right) \times CaO 2 \left(ml/dl\right) \times 10 \left(dl/L\right) ,$ (10) wherein CaO2 is arterial oxygen content (ml/dl) defined by the following formula: $CaO 2 \left(ml/dl\right) = Hb \left(g/dl\right) \times 1.34 \left(ml {O}_{2}/g Hb\right) \times {SaO}_{2} \left(%\right) + {PaO}_{2} \left(mm Hg\right) \times 0.003 \left(ml {O}_{2}/dl\cdot mm Hg\right) .$ (11) Thus, the calculation of oxygen delivery during cardiopulmonary bypass is dependent upon the patient's hemoglobin level during the procedure, which may be measured using a Hb sensor, or derived from measured HCT from HCT sensor 34, or which may be calculated by the fluids circuit 54. SaO₂ is the arterial Hb oxygen saturation (%), which during cardiopulmonary bypass procedures is approximately 99-100%. In an embodiment, instead of assuming arterial Hb oxygen saturation is 99-100%, the arterial side of the cardiopulmonary bypass system 10 is provided with an oxygen saturation sensor that may be part of the arterial blood sensor 29, and that inputs SaO₂ (%) data into the oxygen/CO₂ circuit 56.

In an embodiment, the arterial blood sensor 29 measures at least the arterial oxygen tension PaO₂ (mm Hg), and inputs measured PaO₂ data into the oxygen/CO₂ circuit 56. In an embodiment, the arterial blood sensor 29 includes sensor components that measure the arterial oxygen tension PaO₂ (mm Hg) and the arterial SaO₂, and inputs measured PaO₂ data and measured SaO₂ into the oxygen/CO₂ circuit 56. In an embodiment, the venous blood sensor 28 measures at least the mixed venous oxygen tension PvO₂ (mm Hg), and inputs measured PvO₂ data into the oxygen/CO₂ circuit 56. In an embodiment, the venous blood sensor 28 includes sensor components that measure the venous oxygen tension PvO₂ (mm Hg) and the venous Hb oxygen saturation (SvO₂, %) and inputs measured PvO₂ data and measured SvO₂ into the oxygen/CO₂ circuit 56. In an embodiment, the venous blood sensor 28 measures SvO₂ but not PvO₂ because PvO₂, which represents dissolved oxygen, constitutes a minor component of venous oxygen content CvO2 (ml/dl) similar to the case of CaO2 in equation (11) above. Dissolved venous oxygen may be ignored then, except in those cases in which the patient becomes profoundly anemic.

Thus, oxygen delivery (DO2, ml/min) to the patient is calculated by the oxygen/CO₂ circuit 56 from dynamic data, including arterial pump flow Qp, arterial Hb oxygen saturation SaO₂, and arterial oxygen tension PaO₂, which are sensor-derived data, and from intra-CPB Hb, which may be sensor-derived data or a value calculated by the fluids circuit 54. The oxygen/CO₂ circuit 56 may calculate an indexed oxygen delivery (DO2i, ml/min/m²), which is indexed to the patient's BSA. In other words, the oxygen/CO₂ circuit 56 may divide the calculated oxygen delivery DO2 (ml/min) by the patient's BSA calculated by the BSA circuit 52, wherein the calculated BSA has been inputted from the BSA circuit 52 to the oxygen/CO₂ circuit 56, to generate an indexed oxygen delivery (DO2i) value. The oxygen/CO₂ circuit 56 then outputs the calculated oxygen delivery DO2, and/or the calculated indexed oxygen delivery DO2i, to the display assembly 48 for automatic and continuous display and/or automatic and intermittent display. Likewise, the values of arterial pump flow Qp, arterial Hb oxygen saturation SaO₂, arterial oxygen tension PaO₂, and intra-CPB Hb, may be transmitted to the display assembly 48 for continuous, or intermittent, display. In accordance with an embodiment of this disclosure, the values of arterial pump flow Qp, arterial Hb oxygen saturation SaO₂, arterial oxygen tension PaO₂, intra-CPB Hb, calculated oxygen delivery DO2, and calculated indexed oxygen delivery DO2i, are transmitted to the memory assembly 50 for storage and future reference.

For the purposes of this disclosure, a parameter is said to be "indexed" when it represents a parameter weighted by the patient's BSA. For example, indexed oxygen delivery DO2i is equal to oxygen delivery DO2 divided by the BSA (i.e., DO2i = DO2/BSA). As another example, indexed oxygen consumption VO2i is equal to oxygen consumption VO2 divided by the BSA (i.e., VO2i = VO2/BSA), and so on.

The oxygen/CO₂ circuit 56 is also constructed and/or programmed to calculate patient oxygen consumption (VO2, ml/min) during cardiopulmonary bypass based on the following formula: $VO 2 \left(ml/min\right) = Qp \left(L/min\right) \times \left[CaO2 \left(ml/dl\right)-CvO2 \left(ml/dl\right)\right] \times 10 \left(dl/L\right) ,$ (12) wherein CvO2 is venous oxygen content. Venous oxygen content CvO2 is defined by the following formula: $CvO 2 \left(ml/dl\right) = Hb \left(g/dl\right) \times 1.34 \left(ml O2/g Hb\right) \times {SvO}_{2} \left(%\right) + {PvO}_{2} \left(mm Hg\right) \times 0.003 \left(ml {O}_{2}/dl\cdot mm Hg\right) ,$ (13) wherein SvO₂ is the mixed venous Hb oxygen saturation (%), which is measured by a venous oxygen saturation sensor that may be part of the venous blood sensor 28, and that inputs SvO₂ (%) data into the oxygen/CO₂ circuit 56. Mixed venous Hb oxygen saturation SvO₂ is a measure of the amount of oxygen bound to hemoglobin in venous blood returning to the heart-lung machine 10. PvO₂ (mm Hg) is a measure of the partial pressure of dissolved oxygen in the venous blood returning to the heart-lung machine 10, and is measured by the venous blood sensor 28 or a component of the venous blood sensor 28, and then is input as PvO₂ data to the oxygen/CO₂ circuit 56.

Thus, patient oxygen consumption (VO2, ml/min) is calculated by the oxygen/CO₂ circuit 56 from dynamic data, including arterial pump flow Qp, mixed venous Hb oxygen saturation SvO₂, and mixed venous oxygen tension PvO₂, which are sensor-derived data, and from intra-CPB Hb, which may be sensor-derived data or a value calculated by the fluids circuit 54. The oxygen/CO₂ circuit 56 may calculate an indexed oxygen consumption (VO2i, ml/min/m²), which is indexed to the patient's BSA. In other words, the oxygen/CO₂ circuit 56 may divide the calculated oxygen consumption VO2 (ml/min) by the patient's BSA calculated by the BSA circuit 52, which has been inputted from the BSA circuit 52 to the oxygen/CO₂ circuit 56, to generate an indexed oxygen consumption (VO2i) value. The oxygen/CO₂ circuit 56 then outputs the calculated oxygen consumption VO2, and/or the calculated indexed oxygen consumption VO2i, to the display assembly 48 for automatic and continuous display and/or automatic and intermittent display. Likewise, the values of arterial pump flow Qp, mixed venous Hb oxygen saturation SvO₂, mixed venous oxygen tension PvO₂, and intra-CPB Hb, may be transmitted to the display assembly 48 for continuous, or intermittent, display. In accordance with an embodiment of this disclosure, the values of arterial pump flow Qp, mixed venous Hb oxygen saturation SvO₂, mixed venous oxygen tension PvO₂, intra-CPB Hb, calculated oxygen consumption VO2, and calculated indexed oxygen consumption VO2i, are transmitted to the memory assembly 50 for storage and future reference.

The oxygen/CO₂ circuit 56 is also constructed and/or programmed to calculate the ratio of oxygen delivery to oxygen consumption (DO2/VO2). This calculated ratio DO2/VO2 is then outputted by the oxygen/CO₂ circuit 56 to the display assembly 48 for continuous, or intermittent display, and to the memory assembly 50 for storage and future reference. There is no need to ratio the indexed oxygen delivery DO2i to the indexed oxygen consumption VO2i because the ratio DO2/VO2 is equal to the ratio DO2i/VO2i in view of the fact that BSA/BSA = 1.

The oxygen/CO₂ circuit 56 is also constructed and/or programmed to calculate patient carbon dioxide production (VCO2, ml/min) during cardiopulmonary bypass based on the following formula: $VCO 2 \left(ml/min\right) = Qs \left(L/min\right) \times \left[expCO2 \left(mm Hg\right)/760 \left(mm Hg\right)\right] \times 1000 \left(ml/L\right) ,$ (14) wherein expCO₂ is sensor-derived data provided by CO₂ sensor or capnograph 32 pertaining to the partial pressure of carbon dioxide in the exhaust gas from the oxygenator 18, and Qs is the gas flow rate of ventilation gas G (i.e., sweep gas) feeding into oxygenator 18 from a gas source 62. The sweep gas flow rate Qs is set by the perfusionist, and typically is set at the beginning of the cardiopulmonary bypass procedure. In this case, the sweep gas flow rate Qs constitutes static data and the perfusionist may input the value of the gas flow rate Qs to the oxygen/CO₂ circuit 56 using the manual interface portion 42 of interface 40. On the other hand, in accordance with an embodiment of this disclosure, the oxygenator 18 may be provided with gas flow meter 64 to continuously measure the sweep gas flow rate Qs and to input the sweep gas flow rate Qs data to the oxygen/CO₂ circuit 56 via the machine interface portion 44. The oxygen/CO₂ circuit 56 may also calculate an indexed patient carbon dioxide production VCO2i by dividing the patient carbon dioxide production VCO2 by the patient's BSA calculated by the BSA circuit 52.

Thus, patient carbon dioxide production (VCO2, ml/min) is calculated by the oxygen/CO₂ circuit 56 from dynamic data, including exhaled carbon dioxide expCO₂, which is sensor-derived data, and sweep gas flow rate Qs into the oxygenator 18, which is sensor-derived data that may be static or dynamic in nature. The oxygen/CO₂ circuit 56 may calculate an indexed carbon dioxide production (VCO2i, ml/min/m²), which is indexed to the patient's BSA. The oxygen/CO₂ circuit 56 then outputs the calculated carbon dioxide production VCO2, and/or the calculated indexed oxygen consumption VCO2i, to the display assembly 48 for automatic and continuous display and/or automatic and intermittent display. Likewise, the values of exhaled carbon dioxide expCO₂ and ventilation gas flow rate Qs (also known as "blender flow rate," reported in ml/min or L/min) may be transmitted to the display assembly 48 for continuous, or intermittent, display. In accordance with an embodiment of this disclosure, the values of exhaled carbon dioxide expCO₂, ventilation gas flow rate Qs, calculated carbon dioxide production VCO2, and calculated indexed carbon dioxide production VCO2i, are transmitted to the memory assembly 50 for storage and future reference.

The oxygen/CO₂ circuit 56 is also constructed and/or programmed to calculate the ratio of oxygen delivery to carbon dioxide production (DO2/VCO2). This calculated ratio DO2/VCO2 is then outputted by the oxygen/CO₂ circuit 56 to the display assembly 48 for continuous display, or intermittent display, and to the memory assembly 50 for storage and future reference. There is no need to ratio the indexed oxygen delivery DO2i to the indexed carbon dioxide production VCO2i because the ratio DO2/VCO2 is equal to the ratio DO2i/VCO2i in view of the fact that BSA/BSA = 1.

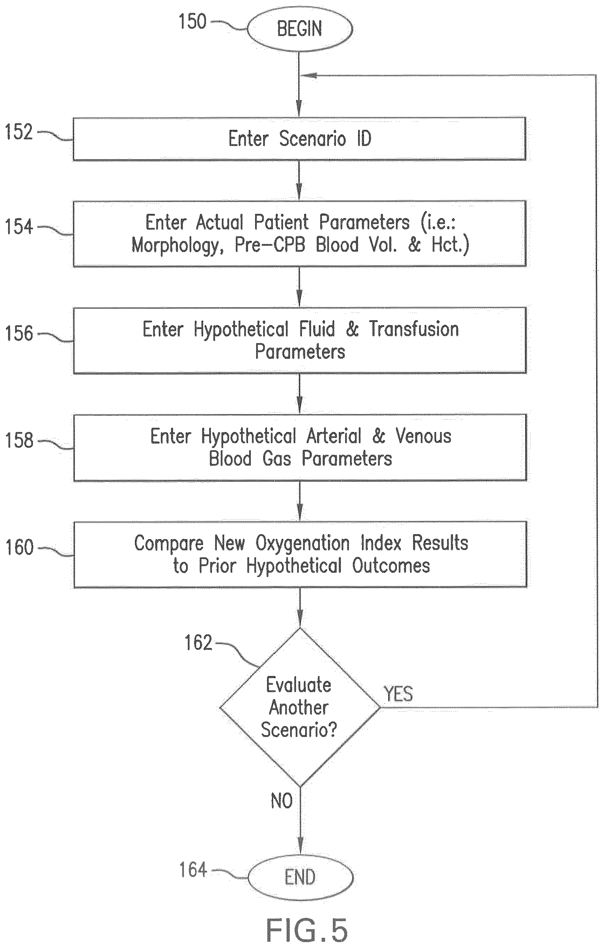

Because calculated oxygen delivery DO2, indexed oxygen delivery DO2i, oxygen consumption VO2, and indexed oxygen consumption VO2i, are functions of intra-CPB Hb and arterial pump flow Qp, the processor 46 is operable to perform simulations, based on hypothetical interventions with respect to pRBC transfusions and/or fluid infusions and/or changes in pump flow, for example, which may cause changes in the patient's oxygen delivery DO2, indexed oxygen delivery DO2i, oxygen consumption VO2, and indexed oxygen consumption VO2i. In addition, because calculated carbon dioxide production VCO2 and indexed carbon dioxide production VCO2i are functions of ventilation gas flow rate Qs and exhaled carbon dioxide expCO₂, the processor is operable to perform simulations based on hypothetical interventions that may alter these parameters, and/or based on estimates and/or guesstimates of how these parameters may fluctuate during the cardiopulmonary bypass procedure. One or more non-limiting examples of the simulation function of the calculation-simulation-monitoring system 12 are provided below with respect to FIGs. 4 and 5.

### SIMULATION EMBODIMENT(S) AND GRAPHICAL USER INTERFACE

FIG. 4 illustrates a non-limiting embodiment of manual interface portion 42 integrated with the display assembly 48 to form a graphical user interface (GUI). The manual interface portion 42 includes a static parameters portion 70 that is used to enter the values of static parameters manually into the calculation-simulation-monitoring system 12. The static parameters portion 70 includes a plurality of data portals 72, each to be populated manually with a particular static parameter value using either a keyboard with touchscreen technology, or other device for entering numerical data. As shown in FIG. 4, the plurality of data portals 72 may include a height data portal 74, a weight data portal 76, a data portal 78 for the patient's pre-CPB blood volume per unit weight, a data portal 80 for the patient's pre-CPB HCT, a data portal 82 for the priming volume, a data portal 84 for the prime off volume, a data portal 86 for the institutional HCT of pRBCs used for transfusion, and a data portal 88 for the hemoglobin oxygen saturation SaO₂. The data portals shown in FIG. 4 of this disclosure are not limited to those shown in FIG. 4. For example, a data portal for the flow rate Qs of ventilation gas may be included in the static parameters portion 70 if the flow rate Qs of the ventilation gas will not be changed during the cardiopulmonary bypass procedure and, therefore, will constitute a static parameter during the CPB procedure. On the other hand, if arterial blood sensor 29 is constructed to include sensor components for measuring arterial Hb oxygen saturation SaO₂, so that this parameter will constitute a dynamic parameter, then the plurality of data portals 72 may be modified so as to not include the data portal 88 for hemoglobin oxygen saturation, and the measured value of arterial Hb oxygen saturation SaO₂ may be automatically transmitted from the arterial blood sensor 29 to the machine interface portion 44 and then to the display assembly 48 for automatic display by a monitored parameter window.

The display assembly 48, and the machine interface portion 44 and the manual interface portion 42 of the graphical user interface of FIG. 2, are integrated as shown in FIG. 4 to provide a monitored parameters portion 90, which includes a plurality of data windows 92 and/or data portals 94. In this context, a data portal is a field on the graphical user interface that is used to manually enter data, and is operably connected to the manual interface portion 42 connected to input data to the processor 46. On the other hand, a data window is a field on the graphical user interface that is populated automatically with data derived from one of the sensors or devices operably connected to input data into the machine interface portion 44 that is connected to input data to the processor 46. Thus, each data portal 94 is populated manually with a particular dynamic parameter that may change at intervals during the cardiopulmonary procedure, and each data window 92 is populated automatically with a particular dynamic parameter that is to be continuously monitored during the cardiopulmonary bypass procedure.

As shown in FIG. 4, the monitored parameters portion 90 may include a data portal 96 for entering the total volume (ml) of pRBCs that have been transfused into the patient. The monitored parameter portion 90 also may include a data portal 98 for entering the total volume (ml) of volume expanding fluids infused into the patient. Because the amount of blood transfused, and the amount of fluids infused, into the patient during the cardiopulmonary bypass procedure may change as the procedure progresses, a user of the calculation-simulation-monitoring system 12 must periodically update these data fields manually whenever the patient receives an additional transfusion and/or an additional infusion.

The monitored parameters portion 90 may also include a data window 100 for arterial pump flow Qp (L/min), a data window 102 for arterial oxygen tension PaO₂ (mm Hg), a data window 104 for venous Hb oxygen saturation (%), a data window 106 for venous oxygen tension PvO₂ (mm Hg), a data window 108 for exhaled carbon dioxide expCO₂ (mm Hg), and a data window 110 for the flow rate Qs of ventilation sweep gas. The data populating each of these windows is sensor-derived, or is derived from a machine (e.g., the roller blood pump), so the data, which is continuously input, may fluctuate continuously in these windows. As a convenience for the user, the graphical user interface is provided with a freeze frame button 112 which, when activated, instructs the processor 46 to freeze the data outputs displayed in the portals and windows of the monitored parameter portion 90 for a brief period of time, such as between 30 seconds and 2 minutes or between 30 seconds and 1 minute. This allows the user more time to digest the displayed output of the monitored parameter portion 90, when desired. Displayed output that has been frozen may be used to autopopulate data fields of a simulator controlled by the processor 46, as will be described below. Of course, it is within the scope of embodiments of this disclosure to modify the period of time during which the data output displayed by the monitored parameters portion 90 is frozen following activation of the freeze frame button 112.

In an embodiment of this disclosure, activating the freeze frame button 112 freezes the display of data in the monitored parameters potion 90 until a subsequent activation of the freeze frame button 112 unfreezes the display of data by the monitored parameters portion 90. For example, clicking the freeze frame button 112 once may result in the processor 46 freezing the data output to the monitored parameters portion 90, and subsequently clicking the freeze frame button 112 again may result in the processor 46 unfreezing the data output to the monitored parameters portion 90 so that the continuous display of monitored data by the monitored parameters portion 90 is resumed.

The monitored parameters portion 90 may include one or two windows 114 for displaying intra-CPB Hb, which may be either sensor-derived data or data calculated by the fluids circuit 54. Consequently, the intra-CPB Hb may be either a measured output or a derived output. Thus, in accordance with an embodiment of this disclosure, the monitored parameters portion 90 includes only one window that displays sensor-derived Hb data values. In accordance with one embodiment of this disclosure, the monitored parameters portion 90 includes only one window that displays the calculated Hb data values calculated by the fluids circuit 54. In accordance with one embodiment of this disclosure, the monitored parameters portion 90 includes two windows 114, wherein one of these windows displays sensor-derived Hb data values and the other window displays the Hb data values calculated by the fluids circuit 54. In this embodiment in which both the sensor-derived Hb data values and fluids circuit-derived Hb data values are displayed in windows 114 side-by-side, the display assembly of the graphical user interface may also be provided with a window to display a statistical correlation value, such as a coefficient of correlation r value or a correlation coefficient r² as are well known in the field of statistics, which is calculated by the statistical circuit 60 that compares and correlates the sensor-derived Hb and/or HCT values with those theoretical actual Hb and/or HCT values calculated by the fluids circuit 54.

The monitored parameters portion 90 may include additional windows for displaying other calculated values or parameters of interest to a perfusionist, nurse, physician, or other user of the system 12. For example, monitored parameters portion 90 may include a window 115 for displaying the indexed delivered oxygen DO2i, and/or a window 116 for displaying the ratio of delivered oxygen to oxygen consumed (DO2/VO2) or the ratio of indexed delivered oxygen to indexed oxygen consumed (DO2i/VO2i). Monitored parameters portion 90 may also include window 118 for displaying the ratio of delivered oxygen to carbon dioxide produced (DO2/VCO2) or the ratio of indexed delivered oxygen to indexed carbon dioxide produced (DO2i/VCO2i). Thus, the monitored parameters portion 90 allows the users of the calculation-simulation-monitoring system 12 to continuously monitor clinically relevant calculated parameters that previously could not be monitored continuously. Having the ability to monitor such calculated parameters continuously has clinical value for those calculated parameters known to correlate with clinical outcomes. Thus, continuously monitored calculated parameters such as DO2/VO2, DO2i/VO2i, DO2/VCO2, DO2i/VCO2i, etc., may be referred to as calculated clinically relevant outcome parameters. The monitored parameters portion 90 may also be provide with a clock 119 for displaying the time so a user monitoring the monitored parameters portion 90 will know the time at which the monitored parameters are displayed.

For example, the ratio DO2i/VCO2i has been correlated to the rise in lactate levels in patients during cardiopulmonary bypass procedures, which is an indicator of tissue hypoxia and poor patient outcomes. Marco Ranucci et al., Anaerobic Metabolism During Cardiopulmonary Bypass: Predictive Value of Carbon Dioxide Derived Parameters. 81 ANNUALS THORACIC SURGERY 2189, 2193-94 (2006). Ideally, the ratio DO2i/VCO2i is maintained above 5 in order to ensure that the oxygen supply to the patient sufficiently exceeds carbon dioxide production so as to avoid tissue hypoxia and lactate acidosis. The calculation-simulation-monitoring system 12 is able to continuously calculate the ratio DO2i/VCO2i and continuously display its value via the display assembly 48 for the purposes of continuously monitoring this ratio.

Preferably, the graphical user interface is implemented as a liquid crystal display (LCD) providing multiple different colored pixels (e.g., red, green and blue) so that the data displayed by various data windows may be displayed in color. For example, in accordance with an embodiment of this disclosure, the data windows 102, 106, 108, 114, 115, 116, 118 are constructed to display values in normal or desired ranges in the color green, and to display values that fall slightly out of the normal or desired ranges in yellow color, and to display values that fall substantially out of the normal or desired ranges in red color. In this way, the color of the displayed data gives additional notification to the user as to the status of the data with respect to normal or desired values (green in color), and slightly deviant values (yellow in color), and to substantially deviant values (red in color). For example, when the displayed value of the ratio DO2i/VCO2i is above 5 in data window 118, then the displayed value is green in color. When the displayed value of the ratio DO2i/VCO2i is between 4 and 5, then the displayed value in data window 118 is yellow in color. When the displayed value of the ratio DO2i/VCO2i is below 4, then the displayed value in window 118 is red in color.

As another example, a preferred range for intra-CPB Hb is 7-8.33 gm/dl (see, e.g., K. Karkouti et. al., Hemodilution During Cardiopulmonary Bypass is an Independent Risk Factor for Acute Renal Failure in Adult Cardiac Surgery. 129 JOURNAL OF THORACIC CARDIOVASCULAR SURGERY 391-400 (2005), abstract), wherein intra-CPB Hb levels higher than this range are associated with a mild increase in risk of acute renal failure, and intra-CPB Hb levels falling below this range carry a substantially greater risk of acute renal failure. Thus, when the intra-CPB Hb value displayed in window 114 is 7-8.33 gm/dl, the value is displayed in green. When the intra-CPB Hb value displayed in window 114 is above 8.33 gm/dl, then the value is displayed in yellow. When the intra-CPB Hb value displayed in window 114 is below 7.0 gm/dl, then the value is displayed in red. It should be appreciated that these examples of color display notification, with respect to the status of a displayed value with respect to a normal or desired value, are merely illustrative and are non-limiting. The described color display notification scheme may be applied to any window displaying monitored data, whether sensor-derived data or data calculated by the processor 46, or one of its component circuits such as the fluids circuit 54 and/or the oxygen/CO₂ circuit 56. In this context, then, monitoring of data may include displaying normative values and threshold alarms in a real-time system that may be stratified based on (i) normal values, (ii) medium priority values or threshold minimum values, and (iii) critical values.

The graphical user interface of FIG. 4 is provided with an exploratory parameters portion 120, which is used to run simulations based on actual data and hypothetical data, wherein the hypothetical data constitutes estimated data and/or guesstimated data. In this context, actual data is data actually collected by sensors and/or other devices constructed to collect data based on a measurement, and may be provided to the processor 46 via either the manual interface portion 42 or the machine interface portion 44. Thus, actual data also includes actual data compiled by a user, and entered manually via the manual interface portion 42, such as the total volume of pRBCs transfused into the patient or the total volume of fluids infused into the patient. Estimated data is hypothetical data based on an estimate based on fact. For instance, if the surgical team is considering a transfusion of 300 ml pRBCs, then data pertaining to this planned transfusion is based on fact and constitutes estimated data. As another example, a patient's historical data may be used as a factual basis to generate estimated data. As shown in FIG. 4, based on the patient's measured PvO₂ values at 16:43 and at 17:15 corresponding to Hb values of 8.2 g/dl and 9.1 g/dl, a user may estimate a PvO₂ value of 40 mm Hg for the hypothetical scenario SCNR # 4, in which the planned blood transfusion is expected to raise the patient's Hb to above 9.4 g/dl.

As another example of estimation, if the temperature of a patient is lowered, an estimated oxygen consumption rate based on the patient's height and weight may be calculated by the user, or by the processor 46 provided with data input from a patient temperature portal (not shown) of the graphical user interface. In this case, the estimated consumption rate would be entered as manually entered data using a VO2 data portal (not shown), wherein the values of the VO2 data is calculated according to the formula reported by Shingi Ninomiya et al., Virtual Patient Simulator for Perfusion Resource Management Drill, 41 JECT 206-212 (2009), namely: $VO 2 = h \left(weight Kg, temperature °C\right) = \left[\left(-0.00001624\right){\left(weight\right)}^{4}+\left(0.036248\right){\left(weight\right)}^{3}-\left(0.27653\right){\left(weight\right)}^{2}+\left(10.834\right)\left(weight\right)\right] \cdot \left[\left(-0.00029\right){\left(temperature\right)}^{3}+\left(0.0284\right){\left(temperature\right)}^{2}-\left(0.8509\right)\left(temperature\right)+8.2832\right] .$ (15)

Using this calculated estimated value for VO2 based on patient weight and temperature, values for PvO₂ may be estimated by processor 46 using formulas (11), (12), and (13) above, assuming no effect of the patient's body temperature on PaO₂, and assuming either SvO₂ does not change or by estimating SvO₂ as a function of PvO₂ based on the hemoglobin saturation curve. The estimated values for the patient's change in PvO₂ based on temperature change may be entered into data portal 134 of the exploratory parameters portion 120. In this way, simulations pertaining to changes in the patient's temperature may be made based on estimated hypothetical data.

Guesstimated data, on the other hand, is hypothetical data that is not based on fact, although it may be based on intuition. For example, if the surgical team believes the patient's carbon dioxide production may increase following a rise in body temperature of 10 degrees, then the team may guestimate that the patient's carbon dioxide production expCO₂ may rise by 10%. Data manually input into data portal 136 of the exploratory parameters portion 120 corresponding to a hypothetical carbon dioxide production expCO₂ that is 10% higher than the previous baseline would constitute guesstimated data, for example.

The exploratory parameters portion 120 includes a scenario ID window 122 that is automatically populated by indication of numerical order. Thus, the first scenario tested by the user is designated "SCNR #1," and the second scenario tested by the user is designated "SCNR #2," and so on. The exploratory parameters portion 120 of the calculation-simulation-monitoring system 12 permits a user to run one or more simulations to test hypothesis and to compare visually the corresponding predicted results of the different simulations based on calculation. While the simulation parameters portion 120 may be limited by space to a predetermined number of scenario ID windows 122, the calculation-simulation-monitoring system 12 may store additional scenarios in the memory assembly 50 for later use or recall. In this way, all tested scenarios may be accessible for review even though only a limited number of scenarios may be displayed at one time by the display assembly 48.

The exploratory parameters portion 120 includes many of the same kinds of data fields as the monitored parameters portion 90. However, the data fields of the exploratory parameters portion 120 may all be data portals so that data must be manually entered into each field. For example, the exploratory parameters portion 120 may include a data portal 124 for entering the volume of pRBCs transfused, or to be transfused, and a data portal 126 for entering the volume of other fluids infused, or to be infused. The simulated parameters portion 120 may further include a data portal 128 for entering arterial pump flow Qp (ml/min) data, a data portal 130 for entering arterial oxygen tension PaO₂ (mm Hg) data, a data portal 132 for entering mixed venous Hb oxygen saturation (%) data, a data portal 134 for entering mixed venous oxygen tension PvO₂ (mm Hg), a data portal 136 for entering exhaled carbon dioxide expCO₂ (mm Hg) data, and a data portal 138 for entering the flow rate Qs of ventilation sweep gas. In each of these data portals, the user has the ability to enter either actual data or hypothetical data depending on the desire of the user.

For example, in a first hypothetical trial of FIG. 4, designated SNCR #1, the data fields 124 to 138 are populated with one or more actual values from the monitored parameters portion 90, although at least one of these values must be replaced manually with a hypothetical value corresponding to a planned or envisioned clinical intervention and/or clinical course progression, and the like. Each of the data fields 124 to 138 may be populated manually by the user as described above. However, as a convenience, in accordance with an embodiment of this disclosure, the monitored parameters portion 90 of the graphical user interface may be provided with a "snapshot" button that, once activated, takes a snapshot of the data fields of the monitored parameters portion 90 and uses the snapped data to populate corresponding data fields of the next available row of the exploratory parameters portion 120. Thus, this embodiment includes a snapshot mechanism that includes a touch screen sensor(s) or button and associated circuitry and embedded system of the processor 46. When the user activates the snapshot button 146, the processor 46 automatically populates the next available entire row of the exploratory parameters portion 120 with the data from the monitored parameters portion 120 at the time the snapshot is taken. The time that the snapshot is taken is recorded in the scenario ID window 122. Thus, according to the non-limiting example of FIG. 4, the rows designated 16:43, 17:15 and 17:49 are populated with all actual data that has been snapped from the monitored parameters portion 120 at these times. The Hb values in windows 140 corresponding to these snapped rows are sensor-derived values measured in real time by the HCT sensor 34 or Hb sensor. The values displayed by windows 141, 142 and 144 are calculated values from the oxygen/CO₂ circuit 56.

On the other hand, each data row in which at least one data field is populated with hypothetical data is designated in the scenario ID window 122 by "SCNR #X," wherein X is populated with the next sequential integer so that the hypothetical scenarios explored are identified in sequence. Thus, as shown in FIG. 4, the rows designated SCNR #1, SCNR #2, SCNR #3 and SCNR #4 are populated in one or more data fields with hypothetical data. As described above, each of the data fields 124-138 may be manually populated by the user with either actual data or hypothetical data, so that at least one data field is populated with hypothetical data. Of course, more than one data field may be populated with hypothetical data. Thus, depending upon the user's desires, one, two, three, several, or all of the data fields 124-138 may be populated with hypothetical data.

As a convenience, the snapshot button 146 may be used to populate actual data in some of the data fields 124-138. More specifically, when the user activates the snapshot button 146 so that the next available row of the exploratory parameters portion 120 is autopopulated with all actual data from the corresponding data fields 96-110 of the monitored parameters portion 90, this autopopulated row will initially be designated by the time of the snapshot. However, the user may manually replace each of the actual data values with hypothetical data values using the data portals 124-138, respectively. Once at least one of the actual values of the snapshot data populating a row designated by the time of the snapshot (XX:XX) is replaced by hypothetical data, the time is automatically replaced in the scenario ID window 122 with the next sequential "SCNR #X" designation. Once this transition from time of snap shot (XX:XX) to next sequential "SCNR #X" has occurred, it cannot be reversed even if the user replaces the hypothetical data with the original actual data. In this way, it is assured that only unadulterated snapshots are designated by time of snapshot, and that all other data rows are designated as scenarios.

As an additional convenience, in accordance with an embodiment of this disclosure, the freeze frame button 112 and the snapshot button 146 may be used together as follows. A user may activate the freeze frame button 112, thereby freezing the data displayed by the monitored parameters portion 90. While the data displayed by the monitored parameters portion 90 is frozen, the user may activate the snapshot button 146 multiple times, thereby autopopulating multiple successive rows of the exploratory parameters portion 120 with identical snapshotted data from the monitored parameters portion 90. Each of these snapshotted rows of data in the exploratory parameters portion 120 will be designated by the same time designation, which is the time corresponding to the freeze of the monitored parameters portion 90. The user may then modify one or more of the data fields 124-138 of these rows of snapshotted data thereby converting them into hypothetical scenarios designated sequentially by the "SCNR #X" designation.

Referring to the non-limiting example of FIG. 4, the row designated "16:43" constitutes a snapshot at 16:43 of the data fields 96-110 and 114, and the corresponding calculated data fields 115, 116 and 118, of the monitored parameters portion 90, which was generated following activation of the snapshot button 146. Thus, the data fields 124-138 and 140 are populated with actual data, and the data fields 141, 142 and 144 are populated with corresponding calculated data. The rows designated "SCNR #1," "SCNR #2" and "SCNR #3" are all snapshots taken at 16:43 of the monitored parameters portion 90 while the monitored parameters portion 90 was frozen following activation of the freeze frame button 112. However, once a user modified the RBC volume data of data portal 124 to reflect a hypothetical pRBC transfusion of 250 ml, the scenario ID was automatically modified by the processor 46 to designate SCNR #1. Once the user subsequently modified the RBC volume data of the data portal 124 of the next row of data (also referred to as a data array) to reflect a hypothetical pRBC transfusion of 500 ml, the scenario ID of this subsequent row was automatically modified by the processor 46 to designate SCNR #2. Furthermore, once the user subsequently modified the RBC volume data of the data portal 124 of the row of data following SCNR #2 to reflect a hypothetical pRBC transfusion of 750 ml, the scenario ID of this still subsequent row was automatically modified by the processor 46 to designate SCNR #3.

Thus, FIG. 4 illustrates how snapshot data may be used to autopopulate multiple data arrays, so that each of these data arrays may be subsequently modified to explore a different clinical intervention scenario. FIG. 4 illustrates how these different exploratory intervention scenarios will be automatically designated by the processor 46 in order to differentiate the hypothetical scenarios from each other and from snapshots autopopulated with only actual data, which are designated by the time of the snapshot. In this way, comparisons between snapshots of all actual data and hypothetical scenarios are facilitated by a user of the system 12.

Referring again to FIG. 4, it can be appreciated how the system 12 may be used to facilitate clinical decision making in real time during a CPB procedure, or during any surgical and/or medical procedure using extracorporeal circulation. For example, the data array of the first row of the exploratory parameters portion 122, which corresponds to a snapshot of the data array of the monitored parameters portion 90 at time T = 16:43, constitutes all actual data in data fields 124-138 and 140, with corresponding calculated values for data fields 141, 142 and 144. The data array of the second row of the exploratory parameters portion 120, designated SCNR #1, includes hypothetical data in data portal 124 and actual data in data portals 126-138. Based on this hypothetical, corresponding to a hypothetical transfusion of 250 pRBC, the patient's calculated Hb value in data window 140, which is calculated by the fluids circuit 54, is expected to rise to 8.8 g/dl from a sensor-derived Hb value of 8.2 g/dl at 16:43. Calculated corresponding changes in additional parameters such as indexed oxygen delivery DO2i, the ratio of indexed oxygen delivery to indexed oxygen consumption (DO2i/VO2i), and the ratio of indexed oxygen delivery to indexed carbon dioxide production (DO2i/VCO2i), are displayed in data windows 141, 142 and 144, respectively.

The data array of the third row of the exploratory parameters portion 120, designated SCNR #2, includes hypothetical data in data portals 124 and 128, and actual data in data portals 126, and 130-138. Based on this hypothetical, corresponding to a hypothetical transfusion of 500 pRBC and an increase in arterial pump flow Qp from 4.0 L/min to 5.0 L/min, the patient's calculated Hb value in data window 140, which is calculated by the fluids circuit 54, is expected to rise to 9.5 g/dl from a sensor-derived Hb value of 8.2 g/dl at 16:43. Calculated corresponding changes in additional parameters such as indexed oxygen delivery DO2i, the ratio of indexed oxygen delivery to indexed oxygen consumption (DO2i/VO2i), and the ratio of indexed oxygen delivery to indexed carbon dioxide production (DO2i/VCO2i), are displayed in data windows 141, 142 and 144, respectively.

The data array of the fourth row of the exploratory parameters portion 120, designated SCNR #3, includes hypothetical data in data portals 124 and 128, and actual data in data portals 126, and 130-138. Based on this hypothetical, corresponding to a hypothetical transfusion of 750 pRBC and an increase in arterial pump flow Qp from 4.0 L/min to 5.0 L/min, the patient's calculated Hb value in data window 140, which is calculated by the fluids circuit 54, is expected to rise to 10.1 g/dl from a sensor-derived Hb value of 8.2 g/dl at 16:43. Calculated corresponding changes in additional parameters such as indexed oxygen delivery DO2i, the ratio of indexed oxygen delivery to indexed oxygen consumption (DO2i/VO2i), and the ratio of indexed oxygen delivery to indexed carbon dioxide production (DO2i/VCO2i), are displayed in data windows 141, 142 and 144, respectively. Thus, the exploratory parameters portion 122 operates as a simulator that allows a user to explore multiple possible clinical interventions for a patient, and use the calculated results to aid in clinical decision making.

As evident from FIG. 4, the user of the system 12 may compare predicted results of various simulated scenarios, such as SCNR #1, SCNR #2 and SCNR #3, which are displayed by the display assembly 48 of the graphical user interface, as an aid to making clinical decisions. In particular, the data matrix configuration, or tabular configuration, of the exploratory parameters portion 120 allows the user to test out multiple hypothetical scenarios in order to determine which hypothetical scenario is predicted to provide the most desired results.

In an embodiment of this disclosure, once all of the data portals 124-138 have been populated, whether with some hypothetical data and some actual data or with all hypothetical data, then the processor 46 automatically calculates results for parameters displayed in results windows 140, 141, 142 and 144, such as calculated intra-CPB Hb in result window 140, and calculated indexed oxygen delivery DO2i in result window 141, and calculated ratio of indexed oxygen delivery to indexed oxygen consumption (DO2i/VO2i) in result window 142, and calculated ratio of indexed oxygen delivery to indexed carbon dioxide production (DO2i/CO2i) in result window 144. Because windows 140, 141, 142 and 144 display calculated hypothetical results, these data fields are constructed as windows rather than portals because they display calculated results and cannot be used to enter data. In an embodiment of this disclosure, a user must activate a run button 148 after populating the data portals 124-138, whether with some hypothetical data and some actual data or with all hypothetical data, in order to cause the processor 46 to calculate results for the parameters displayed in results windows 140, 141, 142 and 144.

After a user explores a series of hypothetical scenarios (e.g., SCNR #1, SCNR #2, SCNR #3), a desired and/or preferred clinical intervention may be implemented by the health care team and, following a period of observation, a follow-up snapshot of actual data may be collected for comparison to the patient's previous condition. In the non-limiting example of FIG. 4, a snapshot of actual data taken at time T = 17:15 from the monitored parameters portion 90 is used to autopopulate the fifth row of data of the exploratory parameters portion 122 with all actual data in data fields 124-138 and 140. Thus, at T = 17:15, the patient has received 500 ml pRBC and the arterial pump flow Qp has been increased to 5.0 L/min, with the result that the patient's sensor-derived Hb level has improved to 9.4 g/dl, and the calculated values for DO2i, DO2i/VO2i, and DO2i/VCO2i have improved to 413 ml/min/m², 3.88 and 5.23, respectively. Because the patient's Hb has improved and because the patient's DO2i/VCO2i ratio has risen above 5, the health care team may choose to continue to monitor the patient's clinical condition using the monitored parameters portion 90 until further intervention is warranted.

For example, as the CPB procedure progresses, the patient may continue to receive intravenous fluids. According to the snapshot of the monitored parameters portion 120 at T = 17:49, displayed sequentially in the sixth row of the exploratory parameters portion 120, the patient has received a total input fluids of 250 ml intravascularly, and this has resulted in a decrease in sensor-derived Hb to 9.1 g/dl and a decrease in the patient's DO2i/VCO2i ratio to 5.06, which is still above 5 but trending downwards. Using the freeze frame button 112 and the snapshot button 146 as described above, the user may autopopulate the data fields of the seventh row of the exploratory parameters portion 120 with actual data from the monitored parameters portion 96 corresponding to T = 17:49, and then modify one or more of the data fields 124-138 to reflect hypothetical data. In this case, the user has modified the data portal 124 to reflect a potential transfusion of an additional 250 ml pRBC, so the total transfusion of pRBC is entered as 750 in the data portal 124. The user, desiring a more accurate hypothetical calculation, may also modify the data portals 132 and 134 to reflect an estimated improvement in SvO₂ and PvO₂ that is expected to occur with the increased Hb level following the additional transfusion of 250 ml pRBCs. The user's basis for this estimate is the patient's previous Hb values and corresponding SvO₂ and PvO₂ values from the previous snapshots at T = 16:43 and T = 17:15. According to the hypothetical scenario of the seventh data array of the exploratory parameters portion 120, the additional transfusion of 250 ml pRBCs is calculated to cause the patient's Hb level to increase to 9.7 g/dl, and that the corresponding changes in the calculated values for DO2i, DO2i/VO2i, and DO2i/VCO2i are expected to rise to 425 ml/min/m², 4.58 and 5.37, respectively.

Thus, FIG. 4 illustrates how the user may employ data from the monitoring parameters portion 90 and the exploratory parameters portion 122 to monitor the patient's condition during the CPB procedure while exploring hypothetical interventions *in-situ* in real time to ascertain theoretical outcomes in order to optimize patient care. In addition, the exploratory parameters portion 122 permits the user to track the patient's condition as the procedure progresses, and as interventions are made, and as the patient's responses to the interventions are chronicled. The system 12 makes it possible to apply clinical simulation *in-situ* during a surgical and/or medical procedure, such as a CPB procedure employing extracorporeal circulation, in a manner never achieved before or even contemplated. The result is that the system 12 makes it possible to take complicated clinical simulation from the limited realm of education (See, e.g. Elizabeth H. Lazzara et al., Eight Critical Factors in Creating and Implementing a Successful Simulation Program, 40 THE JOINT COMMISSION JOURNAL ON QUALITY AND PATIENT SAFETY 21-29 (2014)) and apply it directly to improving patient care *in-situ* and in real time in the operating room or intensive care setting.

With respect to the non-limiting illustrative embodiment of FIG. 4, it is pointed out that in SCNR #1, SCNR #2 and SCNR #3, these hypothetical scenarios explore the effect of different contemplated pRBC transfusion volumes on the patient's calculated Hb level. In these hypothetical scenarios, the effect of the pRBC transfusions on SvO₂ and PvO₂ was not estimated. This creates some degree of error with respect to calculation of certain parameters such as the ratio of indexed delivered oxygen to indexed oxygen consumption (DO2i/VO2i). As evident from the calculated values for DO2i/VO2i for SCNR #1, SCNR #2 and SCNR #3, these values do not appreciably change despite substantial differences in the volume of pRBC transfusion. This result is seen because in these hypothetical scenarios the values of SvO₂ and PvO₂ have been kept constant, which would only happen if the patient's indexed oxygen consumption VO2i were to increase proportionally to the patient's increased indexed oxygen delivery DO2i following the hypothetical transfusions. However, with respect to SCNR #1, SCNR #2 and SCNR #3, the user has no readily apparent factual basis for estimating how the values of SvO₂ and PvO₂ will change for this patient following transfusion. However, with respect to SCNR #4, the user has a factual basis to make such an estimate, namely, the measured SvO₂ and PvO₂ values corresponding to the measured Hb values for the data array snapshots at T = 16:43 and at T = 17:15. In accordance with this disclosure, the user could have guesstimated values for SvO₂ and PvO₂ for each of these scenarios SCNR #1, SCNR #2 and SCNR #3 in an attempt to improve the accuracy of the predicted values for DO2i/VO2i, for example.

With respect to the snapshot taken at T = 17:49 of FIG. 4, the input fluids data portal 126 reads "250" to reflect that the patient has received a 250 ml infusion since the snapshot taken at T = 17:15. It is pointed out that the data for the data portal 126 for this snapshot had to be entered by the user in the data portal 98 of the monitored parameters portion 90 before the snapshot button 146 is activated. This is because if this data were added to some snapshot autopopulating a data array row of the exploratory parameters portion 122, then the processor 46 would construe the change as a hypothetical scenario and would automatically redesignate the snapshot as a scenario using the designation "SCNR #X."

In an embodiment, the graphical user interface may be provided with touch screen capacity so that a user may select the monitored parameters portion 90 field as a whole and then push the "snapshot" button 146, which is also a touchscreen activated button, to activate the autopopulation feature in which the processor 46 autopopulates the data fields of the exploratory parameters portion 120 from the corresponding data fields of the monitored parameters portion 90. Likewise, the freeze frame button 112 may be a touchscreen activated button. Furthermore, while the snapshot button 146 has been described for use in populating a data array of the exploratory parameters portion 122, in an embodiment of this disclosure the touch screen permits the user to touch and select any of the data arrays in order to autopopulate the next sequential row of the exploratory parameters portion 122. For instance, the user may touch the row of the exploratory parameters portion 122 corresponding to SCNR #3, thereby selecting it, which may be indicated by highlighting the selected row on the screen, and then activate the snapshot button 146, thereby causing the processor 46 to autopopulate the data fields 124-138 of the next available row with the data from the data array of SCNR #3. Such an autopopulated row of the exploratory parameters portion 122 would receive the next available "SCNR #X" designation, where X is the next available sequential integer. For example, based on FIG. 4, the next available designation for such a hypothetical would be SCNR #5. The user could then modify any or all of the autopopulated data to create a different hypothetical scenario based on the data from SCNR #3.

In one embodiment, the processor 46 automatically calculates the results used to populate the result windows 140, 141, 142 and 144 as soon as each data portal of a row for a scenario has been populated with data. In another embodiment, the graphical user interface is provided with the "run" button 148 so that the processor 46 calculates the results used to populate the result windows 140, 141, 142 and 144 only after each data portal of a row for a scenario has been populated with data and only after the "run" button 148 has been activated. In this case, activation of the run button 148, the freeze button 112, and the snapshot button 146 may be by touch screen activation, and/or the graphical user interface may be provided with a keyboard, keypad, and/or cursor integrated with the graphical user interface for activating the buttons 148, 112, 146 using a cursor or like means. The keyboard may be used by a user of the system 12 to enter numerical data into the various data portals, and to move a cursor between data portals as is known in the art. In an embodiment, the keyboard is a touchscreen keyboard integrated into the graphical user interface comprising an LCD screen. In an embodiment, the keyboard is a separate device operably connected with the graphical user interface via electronic circuitry. In an embodiment, the run, snap and freeze buttons 148, 146, 112are independently operable so that operating of the snap button 146 may occur without first actuating the freeze button 112. However, in other embodiments of this disclosure, the snap button 146 may be actuated after the freeze button 112 so that the snap shot data is frozen data.

In one embodiment of this disclosure, the exploratory parameters portion 120 is able to display two or more rows of data, which includes at least one simulated row including one or more hypothetical data values, or at least one snapshot row of actual data, or both at least one simulated row including one or more hypothetical data values and at least one snapshot row of actual data. In one embodiment, the exploratory parameters portion 120 is able to display several rows of data, wherein one or more rows is a simulation row including one or more hypothetical data values and/or one or more rows is a snapshot row of actual data. In one embodiment, the exploratory parameters portion 120 is able to display a plurality of rows of data, whether all rows are simulation rows including one or more hypothetical data values, or whether all rows are snapshot rows of actual data, or whether there is a mixture of simulation rows including one or more hypothetical data values and snapshot rows of actual data. In this embodiment, the exploratory parameters portion 120 is provided with a scrolling feature so that all of the plurality of rows of data are viewable by scrolling the rows through the visible portion of the exploratory parameters portion 120, wherein the remaining portion of the exploratory parameters portion 120, which is not currently visible, is stored in the memory assembly 50 until scrolled into view. In addition, the exploratory parameters portion 120 may be provided with additional display space, or with an additional display monitor, so that the data displayed in tabular form, or as a data matrix, may also be displayed as graphs over time to highlight trends for the user.

### Method Embodiment(s)

In accordance with a first non-limiting method embodiment, a calculation-simulation-monitoring method is provided for calculating, simulating and/or monitoring oxygen delivery, or oxygen consumption, or oxygen delivery and oxygen consumption, for a patient, wherein the method includes the steps of: (a) inputting data via an interface configured to receive data input, wherein the inputted data pertains to one or more input parameters selected from the group consisting of patient input parameters, perfusion input parameters, oxygen delivery input parameters, oxygen consumption input parameters, and carbon dioxide production input parameters; (b) calculating one or more output values based on the one or more input parameters using a processor operably connected to receive data signals from the interface corresponding to the one or more input parameters, wherein the one or more output values are selected from the group consisting of one or more patient morphology values, one or more vascular fluids values, one or more oxygen delivery values, one or more oxygen consumption values and one or more carbon dioxide production values; and (c) displaying the one or more output values calculated by the processor using a monitor display assembly, wherein the monitor display assembly includes a display configured for monitoring at least one of the one or more output values calculated by the processor. In a second non-limiting illustrative method embodiment, the first non-limiting method embodiment is modified so that some data inputted via the interface is manually inputted via a manual interface portion of the interface and some data inputted via the interface is sensor-derived data inputted via a machine interface portion of the interface.

In accordance with a third non-limiting illustrative method embodiment, the first and second non-limiting method embodiments are further modified so that the patient input parameters include a patient's height and the patient's weight, and the at least one patient morphology value includes a body surface area value for the patient. In accordance with a fourth non-limiting illustrative method embodiment, the first, second and third non-limiting method embodiments are further modified so that the perfusion input parameters include a patient's pre-cardiac bypass blood volume per unit weight, the patient's pre-cardiac bypass hematocrit, a priming volume, and a prime-off volume. In accordance with a fifth non-limiting illustrative method embodiment, the first, second, third and fourth non-limiting method embodiments are further modified so that the perfusion input parameters further include a hematocrit of packed red blood cells, a volume of a packed red blood cell infusion, and a volume of a fresh frozen plasma infusion and/or of volume expanding fluids, and the at least one vascular fluids value includes an intra-cardiac bypass hematocrit value or an intra-cardiac bypass hemoglobin value.

In accordance with a sixth non-limiting illustrative method embodiment, the first, second, third, fourth and fifth non-limiting method embodiments are modified so that the oxygen delivery input parameters include an SaO₂ and a PaO₂, and the oxygen consumption input parameters include an SvO₂, and a PvO₂. In accordance with a seventh non-limiting illustrative method embodiment, the first, second, third, fourth, fifth and sixth non-limiting embodiments are further modified so that at least one oxygen delivery value is a function of cardiac bypass pump flow, the SaO₂ and the PaO₂, and at least one oxygen consumption value is a function of the cardiac bypass pump flow, the SvO₂, and the PvO₂, and the processor also calculates a ratio of oxygen delivery (DO₂) to oxygen consumption (VO₂), or a ratio of indexed oxygen delivery (DO₂ᵢ) to indexed oxygen consumption (VO₂ᵢ), or both ratios.

In accordance with an eighth non-limiting illustrative method embodiment, the first, second, third, fourth, fifth, sixth and seventh non-limiting method embodiments are modified so that the carbon dioxide production input parameters include an expired CO₂ and a sweep gas flow (Qs) of an oxygenator of a heart-lung machine. In accordance with a ninth non-limiting illustrative embodiment, the first, second, third, fourth, fifth, sixth, seventh and eighth non-limiting embodiments are further modified so that at least one carbon dioxide production value is a function of the expired CO₂ and the sweep gas flow (Qs) of an oxygenator of a heart-lung machine, and the processor also calculates a ratio of oxygen delivery (DO₂) to carbon dioxide production (VCO₂), or a ratio of indexed oxygen delivery (DO₂ᵢ) to indexed carbon dioxide production (VCO₂ᵢ), or both the ratio of oxygen delivery (DO₂) to carbon dioxide production (VCO₂) and the ratio of indexed oxygen delivery (DO₂ᵢ) to indexed carbon dioxide production (VCO₂ᵢ).

In accordance with a tenth non-limiting illustrative method embodiment, the first, second, third, fourth, fifth, sixth, seventh, eighth and ninth non-limiting embodiments are modified so that the interface includes a manual input portion configured for manual input of at least one of the one or more input parameters by a user. In accordance with an eleventh non-limiting illustrative embodiment, the first, second, third, fourth, fifth, sixth, seventh, eighth, ninth and tenth non-limiting embodiments are further modified so that the manual input portion is configured for manual input of a plurality of input parameters by the user, wherein some of the input parameters are real data inputs and some of the input parameters are hypothetical data inputs. In accordance with a twelfth non-limiting illustrative embodiment, the first, second, third, fourth, fifth, sixth, seventh, eighth, ninth, tenth and eleventh non-limiting embodiments are further modified so that the one or more output values calculated by the processor include at least one real output value and at least one predicted outcome output value that are both displayed on the display, wherein the at least one predicted outcome output value corresponds to a clinical simulation.

Thus, in accordance with one or more method embodiments disclosed herein, real (actual) and hypothetical data entered manually may be combined in calculations with sensor-derived data in order to determined real output values for clinical parameters corresponding to a patient as well as to determine in real time (i.e., during the course of a cardiopulmonary bypass operation) and *in-situ* hypothetical predicted outcome output values based on clinical simulations. In this way, actual information pertaining to the patient's clinical status may be collected and monitored during a cardiopulmonary bypass procedure at the same time as hypothetical information is calculated relating to clinical simulation(s). The selection of hypothetical information is based on monitored data collected in real time during a surgical and/or medical procedure, such as during the course of a cardiopulmonary bypass operation or during another kind of extracorporeal bypass circulation procedure. The combination of sensor-derived data and calculated data may guide the surgical/medical team with respect to optimizing clinical management of the patient during the cardiopulmonary bypass procedure, or extracorporeal bypass circulation procedure, so as to minimize morbidity and mortality following cardiopulmonary bypass procedures and other extracorporeal bypass circulation procedures.

FIG. 5 provides a flow diagram with respect to an embodiment of a simulation method disclosed herein. According to the flow diagram of FIG. 5, a graphical user interface is in a ready mode at a first step 150. Such a graphical user interface may be one constructed to have the features of the graphical user interface of FIG. 4. In a second step 152, a user enters a scenario ID into the data field 122 of the simulated parameters portion 120, which may be referred to as simulator 120. Subsequently, in step 154, actual patient parameters are entered into the appropriate data portals of the static parameters portion 70 and the simulator 120. For example, patient parameters such as height and weight are entered manually into data portals 74 and 76, respectively. Other parameters, such as pre-CPB blood volume per unit weight and pre-CPB HCT are entered manually in data portals 78 and 80, respectively, and so on, until all static and dynamic parameters pertaining to actual data have been entered. In this step 154, the data portals are populated with manually inputted data, except when a snapshot of actual data may be used appropriately to populate certain data fields of the simulator with sensor-derived data, or with previously manually entered data. When data is autopopulated into the data portals 124, 126, 128, 130, 132, 134, 136 and 138, the source of data for these data portals and data windows are corresponding data fields 96, 98, 100, 102, 104, 106, 108, and 110, respectively, of the monitored parameters portion 90, which are continuously monitored data fields.

In step 156, hypothetical fluid and transfusion parameters may be entered via data portals of the simulator 120 so that simulations with respect to pRBC transfusion and/or fluid infusion and/or changes in cardiac pump speed may be explored, and their predicted effect on calculated clinically relevant outcome parameters determined. In step 158, hypothetical data with respect to arterial and venous blood gas parameters, such as SaO₂, PaO₂, SvO₂, and PvO₂, as well as hypothetical data with respect to parameters pertaining to ventilation, such as expCO₂ and Qs, may be input into the appropriate data portals of the simulated parameters portion 120, so that simulations with respect to changes in blood gas parameters and/or ventilation parameters can be run, and their predicted effect on calculated clinically relevant outcome parameters determined. While steps 156 and 158 are shown as separate steps, these two steps may be combined in an embodiment of this disclosure. In step 160, the results of running multiple simulations are compared. For example, the calculated oxygenation and carbon dioxide production variables, indexed to the patient's BSA, may be compared as ratios for the various different hypothetical scenarios, such as shown in FIG. 4 with respect to indexed ratios such as DO2i/VO2i and DO2i/VCO2i. Of course, in other embodiments of this disclosure, calculated values for DO2, VO2 and VCO2, and/or DO2i, VO2i and VCO2i, may be displayed by the display assembly 48 as part of step 160 so these values may be compared visually without resorting to ratios.

In step 162, the simulator method permits the user to initiate another scenario for simulation, thereby recycling back to step 152. In this case, the method repeats steps 152 to 160. If at step 162 the user chooses not to initiate another scenario, then the simulator 120 may remain in a ready mode, as in step 150.

In accordance with another method embodiment of this disclosure, a calculation-simulation-monitoring method for calculating, simulating and/or monitoring one or more clinically relevant parameters for a patient *in situ* during patient monitoring is provided, wherein at least one of these clinically relevant parameters is a function of multiple data input parameters, wherein the method includes the steps of: (a) inputting data via an interface configured to receive data input for multiple input parameters, wherein some data inputted via the interface is patient data manually inputted via a manual interface portion of the interface, and a plurality of patient monitoring sensors are operably connected to input data to a machine interface portion of the interface so that some data inputted via the interface is sensor-derived data inputted via the machine interface portion, and wherein the interface further comprises a snapshot mechanism; (b) displaying values of the multiple input parameters on a monitor display assembly, wherein the multiple input parameters include the manually inputted patient data and the sensor-derived inputted data; (c) activating the snapshot mechanism to autopopulate displayed values of the multiple input parameters into a simulator portal; (d) modifying at least one of the autopopulated values of the simulator portal so as to provide hypothetical data input to a processor, then employing the processor to calculate at least one predicted outcome output value corresponding to a clinical simulation based on the hypothetical data input; and (e) displaying the at least one predicted outcome output value calculated by the processor on the monitor display assembly. The sensors employed by this method may include any or all of the sensors of FIG. 1B. The snapshot mechanism may be selectively activated by a perfusionist, clinician, or other health care provided, to autopopulate the displayed values with multiple input parameters selected at a particular time during a medical and/or surgical procedure, such as during a cardiopulmonary bypass procedure or other extracorporeal circulation bypass procedure (e.g., MECC procedures, ECMO procedures, PALP procedures, and dialysis).

In an embodiment, this calculation-simulation-monitoring method may be further modified to include the step (f) of initiating a clinical intervention based on the at least one predicted outcome output value. In another embodiment of this disclosure, the calculation-simulation-monitoring method may be modified instead to include the steps of (f) calculating one or more clinically relevant output values based on the inputted data that comprises the multiple input parameters, wherein the processor performs the calculation of the one or more clinically relevant output values; and (g) displaying the one or more clinically relevant output values calculated by the processor on the monitor display assembly. This modified method may be further modified to include the step (h) of initiating a clinical intervention based on the one or more clinically relevant output values, which may be further modified so that the clinical intervention is initiated based on the one or more clinically relevant outcome values and on the at least one predicted outcome value. According to this disclosure, a clinical intervention includes any of the clinical interventions described herein, and combinations thereof, including but not limited to packed RBC transfusion, infusion of fluids, and changing operating parameters of the extracorporeal circulation system such as arterial pump speed Qp, flow rate Qs of ventilation gas, etc., or any intervention reasonably known or believed by the clinician to affect a clinically relevant outcome parameter for the patient.

### Monitor Embodiment(s)

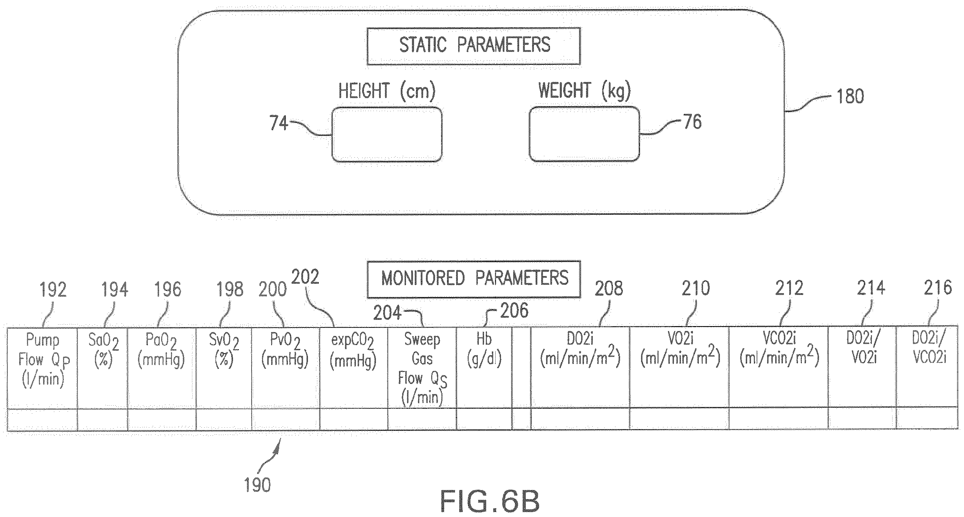

In accordance with an embodiment, as shown in FIGs. 6A and 6B, an oxygen delivery and consumption calculation and monitoring system 168 is provided that is operable to continuously calculate and monitor one or more clinically relevant outcome parameters pertaining to oxygen delivery, or oxygen consumption, or oxygen delivery and oxygen consumption, for a patient, wherein the system 168 is connected to venous blood sensor 28, to arterial blood sensor 29, to flowmeter 30 (or to an RPM-based flow rate measuring system of a roller pump if the cardiac pump is a roller pump), to CO₂ sensor or capnograph 32, to HCT sensor 34 (or a Hb sensor), and to sweep gas flow meter 64, in the same manner as the calculation-simulation-monitoring system 12, as shown in FIG. 1B. Thus, the venous blood sensor 28 is connected to machine interface portion 44 so as to input SvO₂ and PvO₂ to the processor 170 of the oxygen delivery and consumption calculation and monitoring system 168, wherein the venous blood sensor 28 is constructed with sensor components to measure SvO₂ and with sensor components to measure PvO₂. The arterial blood sensor 29 is connected to machine interface portion 44 so as to input SaO₂ and PaO₂ to the processor 170 of the oxygen delivery and consumption calculation and monitoring system 168, wherein the arterial blood sensor 29 is constructed with sensor components to measure SaO₂ and with sensor components to measure PaO₂.

The system 168 is also connected to flowmeter 30 (or to a flow rate measuring system of a roller pump if the cardiac pump is a roller pump) via machine interface portion 44 so that data pertaining to arterial pump speed Qp is continuously input into the processor 170. The system 168 is connected to CO₂ sensor or capnograph 32 via machine interface portion 44 so that data pertaining to carbon dioxide exhausted from the oxygenator 18 is continuously input into the processor 170. The system 168 is connected to HCT sensor 34 (or a Hb sensor) via machine interface portion 44 so that data pertaining to the patient's hematocrit and/or hemoglobin are continuously input into the processor 170. The system 168 is connected to sweep gas flow meter 64 via machine interface portion 44 so that data pertaining to the rate of flow of ventilation gas Qs supplied to the oxygenator 18 are continuously input into the processor 170.

The processor 170 is constructed and/or programmed to calculate one or more clinically relevant outcome parameters pertaining to oxygen delivery, or oxygen consumption, or oxygen delivery and oxygen consumption, for a patient based on the various data input from sensors 28, 29, 30, 32, 34 and 64. Using the formulas (10), (11), (12), (13) and (14), the processor 170 calculates and outputs values in a continuous fashion pertaining to oxygen delivery DO2, oxygen consumption VO2, and carbon dioxide production VCO2. Furthermore, the processor 170 may calculate and output values in a continuous fashion pertaining to clinically relevant outcome parameters such as the ratio of oxygen delivery to oxygen consumption, namely, DO2/VO2, and/or the ratio of oxygen delivery to carbon dioxide production, namely, DO2/VCO2. In an embodiment, values indexed to the patient's BSA are provided by the processor 170 because the graphical user interface of the system 168 is provided with data portals 74 and 75 for entering the patient's weight and height, respectively.

In an embodiment, the system 168 is provided with both a display assembly 48 and a memory assembly 50 so that calculated outputs may be both displayed for immediate monitoring purposes and stored for later reference, review and/or retrieval by a health care professional or other user. In an embodiment, the system is provided with a display assembly 48 for immediate monitoring purposes; however, the memory assembly 50 is only sufficient for the purposes of performing calculations. The calculated values are not stored for later review and/or reference by a health care professional or other user, but are only displayed by the display assembly 48 in a transitory fashion for immediate and continuous monitoring purposes. In an embodiment, the memory assembly 50 includes long term memory storage capability and the display assembly 48 includes additional display space, and/or an additional display monitor, so that the calculated outputs may be displayed in graphs over time to highlight for the user trends in the calculated outputs data over time.

As shown in FIGs. 6A and 6B, an embodiment of the graphical user interface of an oxygen delivery and consumption calculation and monitoring system 168 may include a manual interface portion 42 that includes a static parameters portion 180, which includes a data portal 74 for entering height data and a data portal 76 for entering weight data. A keyboard with cursor, keypad, or other data input device, may be provided so a user may enter numerical height data and numerical weight data in the appropriate data portals. The keyboard may be a physically separate device operably connected to the system 168, or it may be a touchscreen keyboard integrated into an electronic graphical user interface, such as an LCD screen. The graphical user interface also includes a monitored parameters portion 190, which includes only data windows. Each data window is automatically populated with sensor-derived data from an appropriate sensor. For example, the data window 192 is populated with cardiac pump flow data from flowmeter 30 (or from a RPM-based flow rate measuring system of a roller pump if the cardiac pump is a roller pump). Data window 194 is populated with SaO₂ data from a sensor component of the arterial blood sensor 29 and data window 196 is populated with PaO₂ data from another sensor component of the arterial blood sensor 29. Data window 198 is populated with SvO₂ data from a sensor component of the venous blood sensor 28 and data window 200 is populated with PvO₂ data from another sensor component of the venous blood sensor 29. Data window 202 is populated with expCO₂ data from CO₂ sensor or capnograph 32. Data window 204 is populated with ventilation gas flow rate Qs data from sweep gas flow meter 64. Data window 206 is populated with HCT data or Hb data provided by, or derived from, HCT sensor 34 (or from a Hb sensor).

The monitored parameters portion 190 includes a plurality of result windows in which calculated result parameters from processor 170 are displayed for continuous monitoring purposes. For example, result window 208 displays calculated oxygen delivery DO2 or indexed oxygen delivery DO2i. Result window 210 displays calculated oxygen consumption VO2 or indexed oxygen consumption VO2i. Result window 212 displays calculated carbon dioxide production VCO2 or indexed carbon dioxide production VCO2i. Result window 214 displays the ratio of oxygen delivery to oxygen consumption, DO2/VO2, and result window 216 displays the ratio of oxygen delivery to carbon dioxide production, DO2/VCO2. The results displayed in windows 214 and 216 also correspond to the indexed ratios DO2i/VO2i and DO2i/VCO2i, respectfully, because ratios cause the indexing to drop out since BSA/BSA = 1. The results displayed in windows 208, 210, 212, 214 and 216 are continuously calculated and continuously displayed, thereby providing clinicians with a system 168 that permits continuous monitoring of one or more clinically relevant outcome parameters, as well as continuous monitoring of the sensor-derived data used to calculate the one or more clinically relevant outcome parameters.

Because system 168 utilizes only sensor-derived data with respect to its source of patient CPB HCT and/or CPB Hb, the processor 170 does not need to include circuitry and/or programming to calculate CPB HCT and/or CPB Hb based on the patient's perfusion parameters relating to blood volume, volume of fluids infused, etc. Processor 170 also does not include circuitry and/or programming to perform simulations because the system 168 does not perform clinical simulations as does system 12.

In an embodiment, system 168 employs an LCD display as a component of the display assembly 48 of the graphical user interface so that sensor-derived data displayed by the data windows and calculated result values displayed by the result windows are displayed in color. With this construction, sensor-derived data and calculated result values falling within respective normal ranges may be displayed in one color, for example green, whereas sensor-derived data displayed by the data windows and calculated results values falling outside the normal range but within a first range of interest (i.e., a first deviant range) are displayed in a second color, such as yellow for example. Furthermore, sensor-derived data and calculated results values falling outside the normal range and outside the first range of interest are displayed in a third color, such as red, to indicate further deviation from normal (i.e., a second deviant range). In this way, the system 168 performs a monitoring function and a warning function with respect to normal, abnormal and progressively deviant sensor-derived data values and calculated results values pertaining to clinically relevant outcome parameters that are determined from the sensor-derived data input.

While one or more embodiments pertaining to an oxygen delivery and consumption calculation-simulation-monitoring system have been described, the disclosure is not limited to such systems. For example, the system illustrated in Figures 1B and 3 pertains to an oxygen delivery and consumption calculation-simulation-monitoring system; however, in accordance with an embodiment of this disclosure, the system may be modified to employ only the BSA calculations circuit 52 and the fluids calculations circuit 54, and the corresponding inputs to these circuits, so that the system constitutes a perfusion calculation-simulation-monitoring system operable to perform one or more functions directed to calculating, simulating and monitoring perfusion parameters for a patient, such as intra-CPB hemoglobin. Such a perfusion calculation-simulation-monitoring system may include: an interface configured to receive data input pertaining to one or more input parameters selected from the group consisting of patient input parameters and perfusion input parameters; a processor operably connected to receive data signals from the interface corresponding to the received data input pertaining to the one or more input parameters, wherein the processor calculates one or more output values based on the one or more input parameters, wherein the one or more output values are selected from the group consisting of at least one patient morphology value and at least one vascular fluids value; and a monitor display assembly operably connected to receive the one or more output values calculated by the processor, wherein the monitor display assembly includes a display configured for monitoring at least one of the one or more output values calculated by the processor.

According to an embodiment of this perfusion calculation-simulation-monitoring system, some of the input parameters are real data inputs and some of the input parameters are hypothetical data inputs, and the one or more output values calculated by the processor include at least one predicted outcome output value corresponding to a clinical simulation, wherein the at least one predicted outcome output value is displayed on the display. The processor of the perfusion calculation-simulation-monitoring system may also calculate at least one real output value, which is also displayed on the display with the at least one predicted outcome output value so that the at least one real output value may be monitored, and compared with the at least one predicted outcome value. In an embodiment of the perfusion calculation-simulation-monitoring system, the at least one predicted outcome output value is a patient's intra-CPB hemoglobin or hematocrit.

More broadly, in accordance with an embodiment of this disclosure, a clinical parameter calculation-simulation-monitoring system is provided that is operable to perform one or more functions directed to calculating, simulating and monitoring one or more clinical parameters for a patient, wherein each of these clinical parameters are functions of multiple data input parameters. This clinical parameter calculation-simulation-monitoring system may include: an interface configured to receive data input pertaining to one or more input parameters; a processor operably connected to receive data signals from the interface corresponding to the received data input pertaining to the one or more input parameters, wherein the processor calculates one or more output values based on the one or more input parameters, wherein the one or more output values pertain to clinically relevant outcome parameters; and a monitor display assembly operably connected to receive the one or more output values calculated by the processor, wherein the monitor display assembly includes a display configured for monitoring at least one of the one or more output values calculated by the processor.

According to an embodiment of this clinical parameter calculation-simulation-monitoring system, some of the input parameters are real data inputs and some of the input parameters are hypothetical data inputs, and the one or more output values calculated by the processor include at least one predicted outcome output value corresponding to a clinical simulation, wherein the at least one predicted outcome output value is displayed on the display. The perfusion calculation-simulation-monitoring system described above may be characterized as a non-limiting example of a clinical parameter calculation-simulation-monitoring because its processor also calculates at least one real output value, which is also displayed on a display with the at least one predicted outcome output value so that the at least one real output value may be monitored, and compared with the at least one predicted outcome value. In this way, the clinical parameter calculation-simulation-monitoring system is able to calculate and monitor clinically relevant outcome parameters that are functions of multiple data input parameters in real time, so the calculated and monitored clinically relevant outcome parameters may be employed by clinicians in clinical decision making in a manner that has not been previously available. Furthermore, the more generally embodied clinical parameter calculation-simulation-monitoring system is able to calculate simulated outcomes with respect to clinically relevant outcome parameters, which are complicated functions of multiple data input parameters, based on exploratory therapeutic interventions so the calculated simulated clinically relevant outcome parameters may be employed by clinicians in clinical decision making in real time in a manner that has not been previously available.

Thus, while the present disclosure includes non-limiting illustrative embodiments of a clinical parameter calculation-simulation-monitoring system that calculates, simulates and monitors clinically relevant outcome parameters, such as the perfusion calculation-simulation-monitoring system that calculates, simulates and monitors intra-CPB hemoglobin or hematocrit, and such as the oxygen delivery and consumption calculation-simulation-monitoring system that calculates, simulates and monitors clinically relevant outcome parameters pertaining to oxygen delivery (DO2 or DO2i), and/or oxygen consumption (VO2 or VO2i), and/or oxygen delivery and oxygen consumption ratios (DO2/VO2, DO2i/VO2i, DO2/VCO2, DO2i/VCO2i), this disclosure is not limited to these particular embodiments. In accordance with this disclosure, a clinical parameter calculation-simulation-monitoring system may be constructed that calculates, simulates and monitors any clinically relevant outcome parameter that is the function of multiple input parameters by modifying the input parameters appropriately that are input to the processor, which then calculates, and performs simulations with respect to, the clinically relevant outcome parameter. In this manner, it becomes possible to calculate, simulate, and monitor clinically relevant outcome parameters, which are complicated functions of multiple input variables, in a manner in real time that has not been possible previously.

While systems and methods have been described with reference to certain embodiments within this disclosure, one of ordinary skill in the art will recognize, that additions, deletions, substitutions and improvements can be made while remaining within the scope of the invention as defined by the appended claims.

### Definitions and Abbreviations

For this disclosure, the following definitions and abbreviations are employed.

Arterial oxygen tension PaO₂ (mm Hg) is the measure of the partial pressure of dissolved oxygen in arterial blood. Normal PaO₂ is 85-100 mm Hg.

Arterial oxygen saturation SaO₂ (%) is the measure of the amount of oxygen bound to hemoglobin in the arterial blood leaving the oxygenator of the heart-lung machine.

Body Surface Area (BSA, m²) is the surface area of a human body, and is an aid used in determining blood flow requirements of a patient in a surgical or critical care setting.

Cardiopulmonary Bypass (CPB) pertains to surgical technologies in which the flow of blood in a patient is diverted, either wholly or in part, from the venous inflow to the heart to the aortic root or left ventricle, so that oxygenation of blood and/or removal of carbon dioxide is performed by an oxygenator of a heart-lung machine instead of by the patient's native lungs. For the purpose of this disclosure, the term cardiopulmonary bypass encompasses in scope extra corporeal membrane oxygenation (ECMO) technologies, which also divert, either wholly or in part, venous inflow to the heart to the arterial circulation of a patient so that oxygenation of blood and/or removal of carbon dioxide is performed by an oxygenator of an ECMO system instead of by the patient's native lungs.

Exhaled carbon dioxide (expCO₂, mm Hg) is the measure of carbon dioxide in the exhaust gas exhaled from the oxygenator.

Hematocrit (HCT, %) is the volumetric percentage (%) of blood that is comprised of red blood cells (RBCs). Normal HCT is about 45% for men and about 40 % for women.

Hemoglobin (Hb, g/dl) is the amount of hemoglobin in blood. Normal is 13.8 gm/dl to 17.2 gm/dl for men and 12.1 gm/dl to 15.1 gm/dl for women.

Mixed venous oxygen tension PvO₂ (mm Hg) is the measure of the partial pressure of dissolved oxygen in venous blood returning to the heart-lung machine. Normal PvO₂ is 35-42 mm Hg.

Mixed venous oxygen saturation SvO₂ (%) is the measure of the amount of oxygen bound to hemoglobin in the venous blood returning to the heart-lung machine. Normal SvO₂ is 60-80%.

Pump flow (Qp, l/min) is the flow of blood provided by the arterial blood pump, whether a centrifugal pump or a roller pump.

Ventilation gas flow rate Qs (l/min) is the flow rate of ventilation gas (also referred to as sweep gas) that enters the oxygenator in order to oxygenate blood, and remove carbon dioxide from blood, in the oxygenator.

## Claims

1. An extracorporeal circulation system (10) comprising:
a venous reservoir (14), a blood pump (16) and an oxygenator (18) connected together via tubing; and
an oxygen delivery and consumption calculation-simulation-monitoring system (12);
wherein the calculation-simulation-monitoring system (12) is operable to perform one or more functions directed to calculating, simulating and monitoring oxygen delivery, or oxygen consumption, or oxygen delivery and oxygen consumption, for a patient during a cardiopulmonary bypass procedure, the system (12) being dedicated to facilitating clinical decision making in real time during the cardiopulmonary bypass procedure, wherein the system (12) comprises:
an interface (40) configured to receive data input pertaining to one or more input parameters selected from the group consisting of patient input parameters, perfusion input parameters, oxygen delivery input parameters, oxygen consumption input parameters, and carbon dioxide production input parameters, wherein the interface includes a manual input portion (42) configured for manual input of a plurality of the one or more input parameters by a user and a machine input portion (44), wherein some of the input parameters are real data inputs and some of the input parameters are hypothetical data inputs;
a plurality of sensors operably connected to input data to the machine input portion (44) of the interface, said sensor-generated data being used to monitor values of predictive indicators of the patient's condition during the cardiopulmonary bypass procedure, wherein the plurality of sensors include a CO2 sensor (32) disposed to measure carbon dioxide exhausted from the oxygenator (18) of the extracorporeal circulation system (10), a venous blood sensor (28) disposed to measure oxygen tension of mixed venous blood from a patient, an arterial blood sensor (29) disposed to measure oxygen tension of arterial blood from the oxygenator (18), and a gas flow meter (30) disposed to measure flow rate of a ventilation gas flowing through the oxygenator (18);
a processor (46) operably connected to receive data signals from the interface (40) corresponding to the received data input pertaining to the one or more input parameters, wherein the processor calculates one or more output values based on the one or more input parameters, wherein the one or more output values are selected from the group consisting of at least one patient morphology value, at least one vascular fluids value, at least one oxygen delivery value, at least one oxygen consumption value and at least one carbon dioxide production value; and
a monitor display assembly (48) operably connected to receive the one or more output values calculated by the processor (46), wherein the monitor display assembly includes a display configured for monitoring at least one of the one or more output values calculated by the processor, wherein the one or more output values calculated by the processor (46) include at least one real output value and at least one predicted outcome output value corresponding to a clinical simulation, wherein both the at least one real output value and the at least one predicted outcome output value are displayed on the display.

2. The system (12) according to claim 1, wherein the patient input parameters include a patient's height and the patient's weight, and the at least one patient morphology value includes a body surface area value for the patient.

3. The system (12) according to claim 1 , wherein the perfusion input parameters include a patient's pre-cardiac bypass blood volume per unit weight, the patient's pre-cardiac bypass hematocrit, a priming volume, and a prime-off volume.

4. The system (12) according to claim 3, wherein the perfusion input parameters further include a hematocrit of packed red blood cells, a volume of a packed red blood cell infusion, and a volume of a fresh frozen plasma infusion and/or a volume expander infusion, and the at least one vascular fluids value includes an intra-cardiac bypass hematocrit value or an intra-cardiac bypass hemoglobin value.

5. The system (12) according to claim 1, wherein the oxygen delivery input parameters include an SaO₂ and a PaO2, and the oxygen consumption input parameters include an SvO₂ and a PvO2.

6. The system (12) according to claim 5, wherein at least one oxygen delivery value is a function of cardiac bypass pump flow, the SaO₂ and the PaO2, and at least one oxygen consumption value is a function of the cardiac bypass pump flow, the SvO2, and the PvO2, and the processor (46) also calculates a ratio of oxygen delivery (DO2) to oxygen consumption (VO2), or a ratio of indexed oxygen delivery (DO2i) to indexed oxygen consumption (VO2i), or both ratios.

7. The system (12) according to claim 1, wherein the carbon dioxide production input parameters include an expired CO2 and a sweep gas flow (Qs) through an oxygenator (18) of the extracorporeal circulation system (10).

8. The system (12) according to claim 7, wherein at least one carbon dioxide production value is a function of the expired CO2 and the sweep gas flow (Qs) through the oxygenator (18) of the extracorporeal circulation system (10), and the processor (46) also calculates a ratio of oxygen delivery (DO2) to carbon dioxide production (VCO2), or a ratio of indexed oxygen delivery (DO2i) to indexed carbon dioxide production (VCO2i), or both the ratio of oxygen delivery (DO2) to carbon dioxide production (VCO2) and the ratio of indexed oxygen delivery (DO2i) to indexed carbon dioxide production (VCO2j).

9. The system (12) according to claim 1, wherein the plurality of sensors further include a hematocrit sensor (34), or a hemoglobin sensor, disposed on an extracorporeal blood flow circuit connected to a patient.

10. The system (12) according to claim 1, wherein the interface (40) further comprises a snapshot mechanism, wherein when activated the snapshot mechanism autopopulates values of multiple input parameters received by the interface into a simulator portal, wherein the simulator portal permits manual modification of at least one of the autopopulated values so as to provide hypothetical data input to the processor (46) prior to the calculation by the processor of the at least one predicted outcome output value corresponding to the clinical simulation.

11. The system (12) according to claim 1, wherein the extracorporeal circulation system (10) is a heart-lung machine, a minimal extracorporeal circulation system, an extracorporeal membrane oxygenation system, or a pump assisted lung protection system.

## Patentansprüche

1. System (10) zur extrakorporalen Zirkulation, umfassend:
einen Behälter (14) für venöses Blut, eine Blutpumpe (16) und einen Oxygenator (18), die über Schläuche miteinander verbunden sind; und
ein System (12) zur Berechnung, Simulation und Überwachung des Sauerstoffangebots und -verbrauchs;
wobei das System (12) zur Berechnung, Simulation und Überwachung betrieben werden kann, um eine oder mehrere Funktionen durchzuführen, die auf die Berechnung, Simulation und Überwachung des Sauerstoffangebots oder des Sauerstoffverbrauchs oder des Sauerstoffangebots und des Sauerstoffverbrauchs für einen Patienten während einer kardiopulmonalen Bypass-Operation ausgerichtet sind, wobei das System (12) dazu bestimmt ist, die klinische Entscheidungsfindung in Echtzeit während der kardiopulmonalen Bypass-Operation zu erleichtern, wobei das System (12) Folgendes umfasst:
eine Schnittstelle (40), die dazu konfiguriert ist, Dateneingaben bezüglich eines oder mehrerer Eingabeparameter zu empfangen, die aus der Gruppe bestehend aus Patienteneingabeparametern, Durchblutungseingabeparametern, Sauerstoffangebot-Eingabeparametern, Sauerstoffverbrauch-Eingabeparametern und Kohlendioxydproduktion-Eingabeparametern ausgewählt sind, wobei die Schnittstelle einen Abschnitt (42) für manuelle Eingaben, der für manuelle Eingaben einer Vielzahl des einen oder der mehreren Eingabeparameter durch einen Benutzer konfiguriert ist, und einen Abschnitt (44) für maschinelle Eingaben beinhaltet, wobei es sich bei einigen der Eingabeparameter um Echtdateneingaben und bei einigen der Eingabeparameter um hypothetische Dateneingaben handelt;
eine Vielzahl von Sensoren, die mit Eingabedaten in den Abschnitt (44) für maschinelle Eingaben der Schnittstelle wirkverbunden ist, wobei die von Sensoren generierten Daten verwendet werden, um Werte prognostischer Indikatoren für den Zustand des Patienten während der kardiopulmonalen Bypass-Operation zu überwachen, wobei die Vielzahl von Sensoren einen CO2-Sensor (32), der zum Messen des von dem Oxygenator (18) des Systems (10) zur extrakorporalen Zirkulation ausgestoßenen Kohlendioxyds angeordnet ist, einen Sensor (28) für venöses Blut, der zum Messen der Sauerstoffspannung von gemischt-venösem Blut von einem Patienten angeordnet ist, einen Sensor (29) für arterielles Blut, der zum Messen der Sauerstoffspannung von arteriellem Blut von dem Oxygenator (18) angeordnet ist, und einen Gasdurchflussmesser (30), der zum Messen der Durchflussrate eines durch den Oxygenator (18) strömenden Belüftungsgases angeordnet ist, beinhaltet;
einen Prozessor (46), der wirkverbunden ist, um Datensignale von der Schnittstelle (40) zu empfangen, die den empfangenen Dateneingaben bezüglich des einen oder der mehreren Parameter entsprechen, wobei der Prozessor auf Grundlage des einen oder der mehreren Eingabeparameter einen oder mehrere Ausgabewerte berechnet, wobei der eine oder die mehreren Ausgabewerte aus der Gruppe bestehend aus mindestens einem Patientenmorphologiewert, mindestens einem Vaskulärflüssigkeitenwert, mindestens einem Sauerstoffangebotswert, mindestens einem Sauerstoffverbrauchswert und mindestens einem Kohlendioxydproduktionswert ausgewählt sind; und
eine Überwachungsanzeigebaugruppe (48), die wirkverbunden ist, um den einen oder die mehreren von dem Prozessor (46) berechneten Ausgabewerte zu empfangen, wobei die Überwachungsanzeigebaugruppe eine Anzeige beinhaltet, die zum Überwachen mindestens eines des einen oder der mehreren von dem Prozessor berechneten Ausgabewerte konfiguriert ist, wobei der eine oder die mehreren von dem Prozessor (46) berechneten Ausgabewerte mindestens einen echten Ausgabewert und mindestens einen Ausgabewert eines prognostizierten Ausgangs, der einer klinischen Simulation entspricht, beinhalten, wobei sowohl der mindestens eine echte Ausgabewert als auch der mindestens eine Ausgabewert eines prognostizierten Ausgangs auf der Anzeige angezeigt werden.

2. System (12) nach Anspruch 1, wobei die Patienteneingabeparameter die Größe eines Patienten und das Gewicht des Patienten beinhalten und der mindestens eine Patientenmorphologiewert einen Körperoberflächenwert für den Patienten beinhaltet.

3. System (12) nach Anspruch 1, wobei die Durchblutungseingabeparameter ein Blutvolumen pro Gewichtseinheit eines Patienten vor dem Herz-Bypass, einen Hämatokritwert des Patienten vor dem Herz-Bypass, ein Füllvolumen und ein Volumen ohne Füllen beinhalten.

4. System (12) nach Anspruch 3, wobei die Durchblutungseingabeparameter ferner einen Hämatokritwert von Erythrozytenkonzentrat, ein Volumen einer Erythrozytenkonzentratinfusion und ein Volumen einer Infusion mit gefrorenem Frischplasma und/oder einer Volumenexpanderinfusion beinhalten und der mindestens eine Vaskulärflüssigkeitenwert einen Hämatokritwert während des Herz-Bypasses oder einen Hämoglobinwert während des Herz-Bypasses beinhaltet.

5. System (12) nach Anspruch 1, wobei die Sauerstoffangebot-Eingabeparameter einen SaO2-Wert und einen PaO2-Wert beinhalten und die Sauerstoffverbrauch-Eingabeparameter einen SvO2-Wert und einen PvO2-Wert beinhalten.

6. System (12) nach Anspruch 5, wobei mindestens ein Sauerstoffangebotswert eine Funktion eines Herz-Bypass-Pumpendurchflusses, des SaO2-Werts und des PaO2-Werts ist und mindestens ein Sauerstoffverbrauchswert eine Funktion des Herz-Bypass-Pumpendurchflusses, des SvO2-Werts und des PvO2-Werts ist und der Prozessor (46) zudem ein Verhältnis des Sauerstoffangebots (DO2) zum Sauerstoffverbrauch (VO2) oder ein Verhältnis eines indexierten Sauerstoffangebots (DO2i) zu einem indexierten Sauerstoffverbrauch (VO2i) oder beide Verhältnisse berechnet.

7. System (12) nach Anspruch 1, wobei die Kohlendioxydproduktion-Eingabeparameter einen Wert des austretenden CO2 und einen Spülgasdurchfluss (Qs) durch einen Oxygenator (18) des Systems (10) zur extrakorporalen Zirkulation beinhalten.

8. System (12) nach Anspruch 7, wobei mindestens ein Kohlendioxydproduktionswert eine Funktion des austretenden CO2 und des Spülgasdurchflusses (Qs) durch den Oxygenator (18) des Systems (10) zur extrakorporalen Zirkulation ist und der Prozessor (46) zudem ein Verhältnis des Sauerstoffangebots (DO2) zur Kohlendioxydproduktion (VCO2) oder ein Verhältnis des indexierten Sauerstoffangebots (DO2i) zu einer indexierten Kohlendioxydproduktion (VCO2i) oder sowohl das Verhältnis des Sauerstoffangebots (DO2) zur Kohlendioxydproduktion (VCO2) als auch das Verhältnis des indexierten Sauerstoffangebots (DO2i) zur indexierten Kohlendioxydproduktion (VCO2j) berechnet.

9. System (12) nach Anspruch 1, wobei die Vielzahl von Sensoren ferner einen Hämatokritsensor (34) oder einen Hämoglobinsensor beinhaltet, der an einem mit einem Patienten verbundenen extrakorporalen Blutkreislauf angeordnet ist.

10. System (12) nach Anspruch 1, wobei die Schnittstelle (40) ferner einen Schnappschussmechanismus umfasst, wobei der Schnappschussmechanismus, wenn er aktiviert ist, automatisch Werte mehrerer Eingabeparameter, die an der Schnittstelle empfangen werden, in ein Simulatorportal einspeist, wobei das Simulatorportal eine manuelle Modifizierung mindestens eines der automatisch eingespeisten Werte ermöglicht, um so hypothetische Dateneingaben in den Prozessor (46) bereitzustellen, bevor der Prozessor den mindestens einen Ausgabewert eines prognostischen Ausgangs berechnet, welcher der klinischen Simulation entspricht.

11. System (12) nach Anspruch 1, wobei es sich bei dem System (10) zur extrakorporalen Zirkulation um eine Herz-Lungen-Maschine, ein minimiertes System zur extrakorporalen Zirkulation, ein System zur extrakorporalen Membranoxygenierung oder ein pumpengestütztes Lungenschutzsystem handelt.

## Revendications

1. Système de circulation extracorporelle (10) comprenant :
un réservoir veineux (14), une pompe à sang (16) et un oxygénateur (18) reliés ensemble via une tubulure ; et
un système de calcul-simulation-surveillance de distribution et de consommation d'oxygène (12) ;
dans lequel le système de calcul-simulation-surveillance (12) est utilisable pour mettre en œuvre une ou plusieurs fonctions prévues pour calculer, simuler et surveiller une distribution d'oxygène ou une consommation d'oxygène ou une distribution d'oxygène et une consommation d'oxygène, pour un patient pendant une intervention de pontage cardiopulmonaire, le système (12) étant dédié à faciliter une prise de décision clinique en temps réel pendant l'intervention de pontage cardiopulmonaire, dans lequel le système (12) comprend :
une interface (40) configurée pour recevoir une entrée de données concernant un ou plusieurs paramètres d'entrée sélectionnés à partir du groupe constitué par des paramètres d'entrée de patient, des paramètres d'entrée de perfusion, des paramètres d'entrée de distribution d'oxygène, des paramètres d'entrée de consommation d'oxygène et des paramètres d'entrée de production de dioxyde de carbone, dans lequel l'interface inclut une partie entrée manuelle (42) configurée pour une entrée manuelle d'une pluralité des un ou plusieurs paramètres d'entrée par un utilisateur et une partie entrée machine (44), dans lequel une partie des paramètres d'entrée sont des entrées de données en temps réel et une partie des paramètres d'entrée sont des entrées de données hypothétiques ;
une pluralité de capteurs reliés de manière opérationnelle à des données d'entrée à la partie entrée machine (44) de l'interface, lesdites données générées par capteurs étant utilisées pour surveiller des valeurs d'indicateurs prédictifs de l'état du patient pendant l'intervention de pontage cardiopulmonaire, dans lequel la pluralité de capteurs incluent un capteur de CO2 (32) disposé pour mesurer du dioxyde de carbone évacué de l'oxygénateur (18) du système de circulation extracorporelle (10), un capteur de sang veineux (28) disposé pour mesurer une tension d'oxygène de sang veineux mêlé provenant d'un patient, un capteur de sang artériel (29) disposé pour mesurer une tension d'oxygène de sang artériel provenant de l'oxygénateur (18) et un débitmètre de gaz (30) disposé pour mesurer le débit d'un gaz de ventilation s'écoulant à travers l'oxygénateur (18) ;
un processeur (46) relié de manière opérationnelle pour recevoir des signaux de données provenant de l'interface (40) correspondant à l'entrée de données reçue concernant les un ou plusieurs paramètres d'entrée, dans lequel le processeur calcule une ou plusieurs valeurs de sortie sur la base des un ou plusieurs paramètres d'entrée, dans lequel les une ou plusieurs valeurs de sortie sont sélectionnées à partir du groupe constitué par au moins une valeur de morphologie de patient, au moins une valeur de fluides vasculaires, au moins une valeur de distribution d'oxygène, au moins une valeur de consommation d'oxygène et au moins une valeur de production de dioxyde de carbone ; et
un ensemble formant dispositif d'affichage de moniteur (48) relié de manière opérationnelle pour recevoir les une ou plusieurs valeurs de sortie calculées par le processeur (46), dans lequel l'ensemble formant dispositif d'affichage de moniteur inclut un dispositif d'affichage configuré pour surveiller au moins l'une des une ou plusieurs valeurs de sortie calculées par le processeur, dans lequel les une ou plusieurs valeurs de sortie calculées par le processeur (46) incluent au moins une valeur de sortie réelle et au moins une valeur de sortie d'issue prédite correspondant à une simulation clinique, dans lequel l'au moins une valeur de sortie réelle et l'au moins une valeur de sortie d'issue prédite sont affichées sur le dispositif d'affichage.

2. Système (12) selon la revendication 1, dans lequel les paramètres d'entrée de patient incluent une taille du patient et le poids du patient, et l'au moins une valeur de morphologie de patient inclut une valeur de superficie de corps pour le patient.

3. Système (12) selon la revendication 1, dans lequel les paramètres d'entrée de perfusion incluent un volume de sang de pontage précardiaque du patient par unité de poids, l'hématocrite de pontage précardiaque du patient, un volume d'amorçage et un volume de désamorçage.

4. Système (12) selon la revendication 3, dans lequel les paramètres d'entrée de perfusion incluent en outre un hématocrite de globules rouges concentrés, un volume d'une perfusion de globules rouges concentrés et un volume de perfusion de plasma congelé frais et/ou un volume de perfusion de dilatateur de volume et l'au moins une valeur de fluides vasculaires inclut une valeur d'hématocrite de pontage intracardiaque ou une valeur d'hémoglobine de pontage intracardiaque.

5. Système (12) selon la revendication 1, dans lequel les paramètres d'entrée de distribution d'oxygène incluent un SaO₂ et un PaO2, et les paramètres d'entrée de consommation d'oxygène incluent un SvO₂ et un Pvo2.

6. Système (12) selon la revendication 5, dans lequel au moins une valeur de distribution d'oxygène est fonction du débit de pompe de pontage cardiaque, le SaO₂ et le PaO2, et au moins une valeur de consommation d'oxygène est fonction du débit de pompe de pontage cardiaque, le SvO₂ et le PvO2, et le processeur (46) calcule également un rapport de la distribution d'oxygène (DO2) à la consommation d'oxygène (VO2), ou un rapport de la distribution d'oxygène indexée (DO2i) à la consommation d'oxygène indexée (VO2i), ou ces deux rapports.

7. Système (12) selon la revendication 1, dans lequel les paramètres d'entrée de production de dioxyde de carbone incluent un CO2 expiré et un débit de gaz de balayage (Qs) à travers un oxygénateur (18) du système de circulation extracorporelle (10).

8. Système (12) selon la revendication 7, dans lequel au moins une valeur de production de dioxyde de carbone est fonction du CO2 expiré et du débit de gaz de balayage (Qs) à travers l'oxygénateur (18) du système de circulation extracorporelle (10), et le processeur (46) calcule également un rapport de la distribution d'oxygène (DO2) à la production de dioxyde de carbone (VCO2), ou un rapport de la distribution d'oxygène indexée (DO2i) à la production de dioxyde de carbone indexée (VCO2i), ou le rapport de la distribution d'oxygène (DO2) à la production de dioxyde de carbone (VCO2) et le rapport de la distribution d'oxygène indexée (DO2i) à la production de dioxyde de carbone indexée (VCO2j).

9. Système (12) selon la revendication 1, dans lequel la pluralité de capteurs comprennent en outre un capteur d'hématocrite (34), ou un capteur d'hémoglobine, disposé sur un circuit de circulation sanguine extracorporelle relié à un patient.

10. Système (12) selon la revendication 1, dans lequel l'interface (40) comprend en outre un mécanisme de copie instantanée, dans lequel lorsqu'il est activé le mécanisme de copie instantanée autoalimente des valeurs de multiples paramètres d'entrée reçus par l'interface dans un portail de simulateur, dans lequel le portail de simulateur permet une modification manuelle d'au moins une des valeurs autoalimentées de façon à fournir une entrée de données hypothétiques au processeur (46) avant le calcul par le processeur de l'au moins une valeur de sortie d'issue prédite correspondant à la simulation clinique.

11. Système (12) selon la revendication 1, dans lequel le système de circulation extracorporelle (10) est une machine cœur-poumon, un système de circulation extracorporelle minimale, un système d'oxygénation de membrane extracorporelle ou un système de protection des poumons assisté par pompe.
